# DEMANDE DE BREVET EUROPEEN

(11) **EP 2 263 995 A1**
(43) Date de publication de la demande: **22.12.2010**
(21) Numéro de dépôt: 10184117.9
(22) Date de dépôt: 16.05.2006
(51) Int. Cl.: C07C 59/72, C07C 259/06, C22B 60/02, C22B 60/04

(54) **Para-tertio-butylcalix[6]arènes portant des fonctions triacides en positions 2, 4 et 6, membranes liquides supportées et matériaux supports les comportant et leurs utilisations**

(30) Priorité: 17.05.2005 FR 0504944
(62) Demande divisionnaire de: 06755491.5
(71) Demandeur: INSTITUT DE RADIOPROTECTION ET DE SURETE NUCLEAIRE (I.R.S.N.), 92260 Fontenay aux Rouses (FR)
(72) Inventeur: Cossonet, Catherine, 91430 Igny (FR); Bouvier, Céline, 92320 Chatillon (FR); Duval, Raphaël, 74890 Brenthonne (FR)
(74) Mandataire: Cabinet Plasseraud

(57) **Abrégé**

L'invention concerne de nouveaux para-tertio-butylcalix[6]arènes de formules (IA) et (IB) portant des fonctions triacides carboxyliques ou triacides hydroxamiques en positions 2,4 et 6, et portant d'autres fonctions en positions 1, 3 et 5, des membranes liquides supportées et des matériaux supports les contenant, ainsi que leurs utilisations.

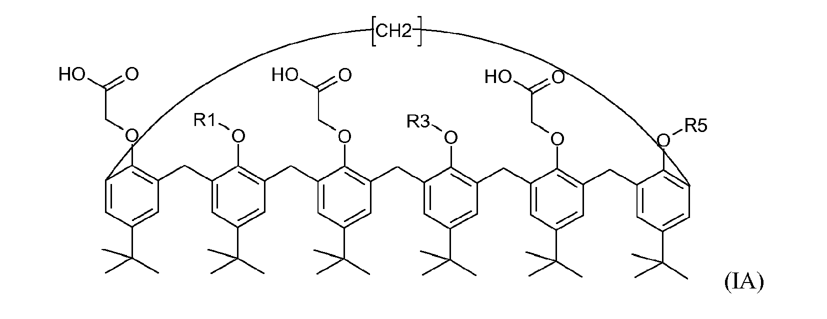

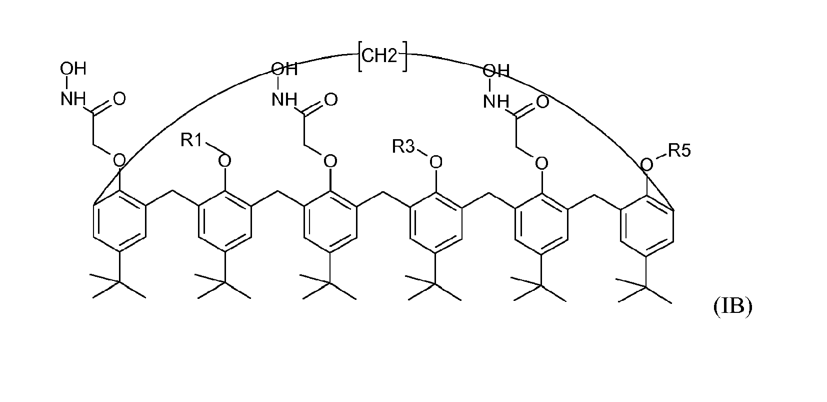

## Description

Au cours du cycle du combustible, de la mine d'uranium aux usines de retraitement, les composés radioactifs sont présents sous des formes physico-chimiques très variées engendrant des toxicités différentes et donc des risques d'exposition du personnel très diversifiés.

Pour assurer la protection de la santé des travailleurs il est nécessaire d'exercer un contrôle médical. Différents examens sont pratiqués sur les travailleurs dont l'analyse des actinides émetteurs alpha éliminés dans les excreta (urines et selles).

Actuellement, la technique analytique de référence est la spectrométrie a. Du fait du faible parcours des particules a dans la matière, il est impossible de mesurer directement l'uranium, l'américium et le plutonium dans l'urine, il est donc nécessaire de fabriquer une source couche mince pour chacun des actinides. Ceci implique une première étape de minéralisation de l'échantillon suivie d'une purification chimique qui permet d'isoler les actinides de la matrice urinaire et de les séparer les uns des autres, afin de limiter les interférences spectrales.

Dans les protocoles actuellement utilisés par tous les laboratoires d'analyses radio-toxicologiques, cette purification repose sur des séparations successives des actinides, à l'aide de colonnes chromatographiques appropriées (Harduin et coll., Radioprotection, 31 n°2, 229-245, 1996). Ces protocoles sont longs (6 jours pour obtenir le résultat final), de l'ordre de 3 jours pour le traitement chimique et 3 jours de comptage par spectrométrie a pour atteindre des niveaux d'activité par isotope inférieurs à 1 mBq.l⁻¹ d'urine.

Dans le contexte d'une évolution technologique constante dans l'industrie nucléaire et de celle de la réglementation, les moyens permettant d'effectuer en routine la surveillance des travailleurs et d'évaluer l'exposition interne des individus en cas d'accident se doivent d'être améliorés.

Il y a donc un besoin réel et crucial en terme de santé à pouvoir disposer dans des délais très courts de résultats des mesures concernant l'uranium, l'américium et le plutonium afin de pouvoir contrer les effets délétères des rayonnements alpha. La mise à disposition de techniques d'analyse plus rapides et plus performantes permettrait également de suivre les émetteurs a dans l'environnement, pour répondre aux problématiques du développement durable.

Afin de résoudre ce problème, de nombreuses études ont été mises en oeuvre mais aucune n'a abouti à ce jour. Les recherches se sont orientées vers des complexants sélectifs des 3 actinides afin de les extraire à partir de matrices complexes comme les milieux biologiques.

La plupart des travaux ont été menés sur des cages complexantes particulières : les calixarènes. Par exemple, en 1993, Araki et coll. ont montré les propriétés complexantes en extraction liquide-liquide, du 1,3,5-O-triméthyl-2,4,6-O-triacide carboxylique-para-tertio-butyl-[calix6] -arène (molécule représentée ci-après par la formule IA), vis-à-vis de l'uranium (Chem. Lett., 829-832, 1993). En 1994, Van Duynoven et coll. ont utilisé la même molécule pour étudier ses équilibres conformationnels (J. Am. Chem. Soc., 116, 5814-5822). En 1997, C. Dinse et coll. (Radioprotection, 32 n°5, 659-671) ont démontré la sélectivité, en extraction liquide-liquide de la molécule de formule A vis-à-vis de l'uranium en présence de plutonium et de sodium.

Plus récemment, un nouvel agent d'extraction, dérivé synthétique de la molécule de formule A, le 1,3,5-O-triméthyl-2,4,6-O-triacide hydroxamique-para-tertio-butyl-[calix6]arène (molécule représentée ci-après par la formule B), a été proposé par Bennoura et coll. dans Journal of Inclusion Phenomena and Macrocyclic Chemistry , 40, 95-98, 2001 pour la complexation de cations.

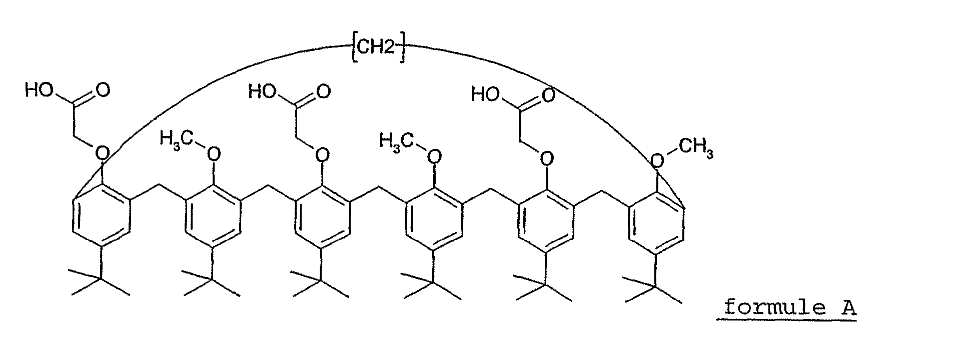

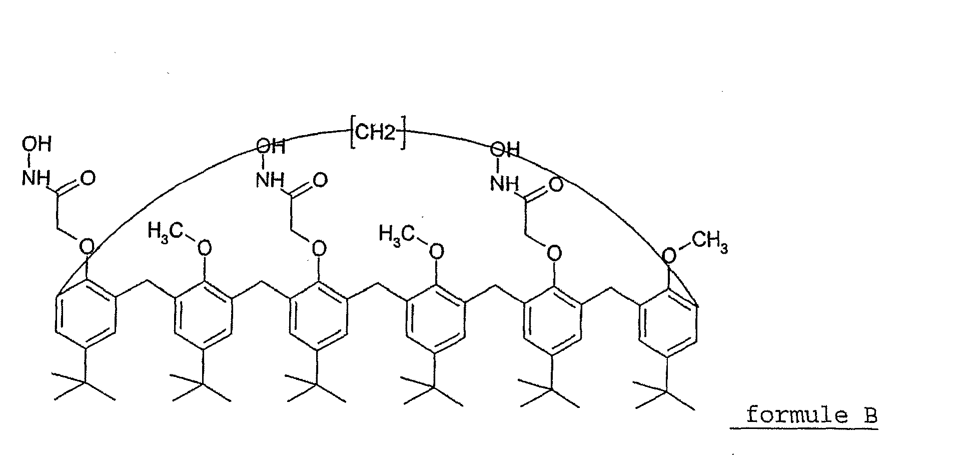

A notre connaissance, les molécules de formules A et B n'ont jamais fait l'objet d'études sur membranes supportées ou sur supports greffés.

L'immobilisation de calixarènes par lien covalent sur des matériaux supports a déjà été décrite par S.P. Alexandratos et coll. dans Macromolecules, 2001, 34, 206-210 puis en 2002 par Trivedi et coll. dans Reactive and Functional Polymers, 50, 205-216. Par ailleurs l'ouvrage de Z. Asfari, édité en 2001 par Kluwer Academic Press (Pays-Bas) et intitulé « Calixarenes », présente dans le chapitre écrit par R. Milbradt et V. Bôhmer, pages 663 à 676, une revue des techniques d'immobilisation et des produits obtenus. Cependant l'immobilisation des calixarènes de formules A et B n'a jamais été décrite.

Aucun des produits ou techniques décrites dans l'art antérieur ne permet l'analyse des cations uranium et/ou américium et/ou plutonium à une teneur de l'ordre de 1 mBq par litre en moins de 6 jours.

De façon surprenante, les inventeurs ont trouvé que le remplacement d'un groupe méthoxy dans les formules A et B décrites dans la littérature, par un autre groupement, et plus avantageusement par le groupement hydroxyle, ne diminuait pas les capacités de complexation vis-à-vis des cations uranium, américium et plutonium.

Cette découverte a été mise à profit pour préparer des membranes liquides supportées contenant les composés de formules générales IA et/ou IB et de nouveaux matériaux supports. Ces membranes et ces matériaux supports présentent la propriété de complexer sélectivement l'uranium et/ou l'américium et/ou le plutonium. La mise en oeuvre de ces membranes supportées dans des colonnes adéquates pour l'analyse par chromatographie d'extraction permet l'analyse des éléments cités précédemment à une teneur de l'ordre de 1 mBq/l.

Ainsi, la présente invention porte sur une nouvelle famille de composés para-tertio-butyl calix[6]arènes de formule (IA) ou (IB) 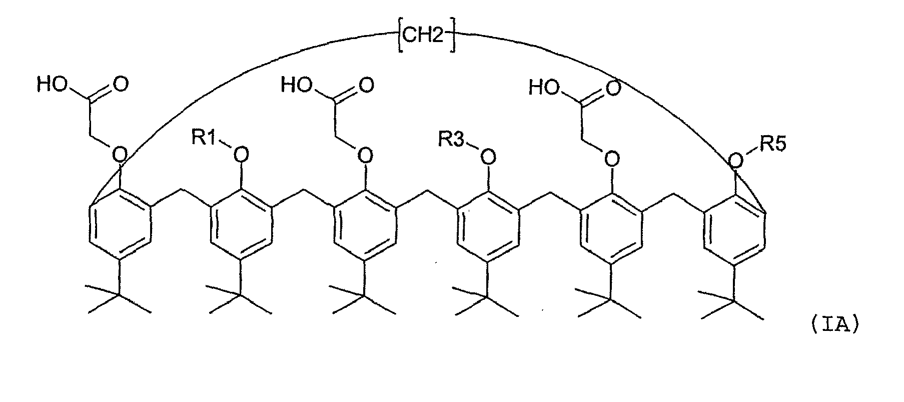 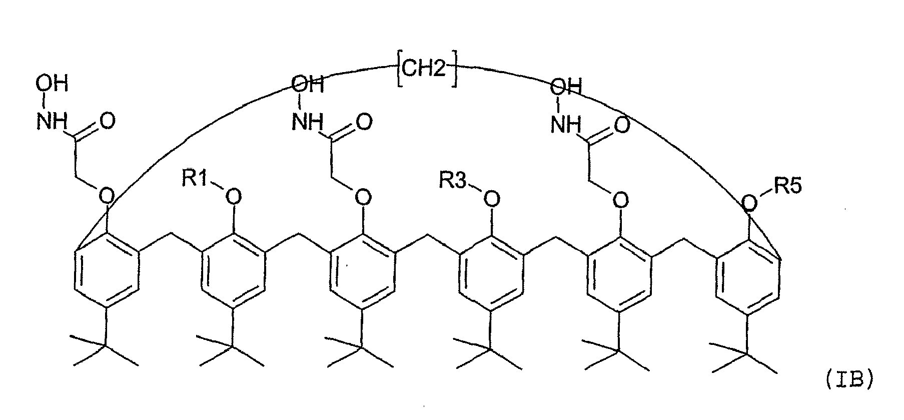 Où R1, R3 et R5, identiques ou différents, représentent, chacun indépendamment :
(i). un atome d'hydrogène, ou d'halogène,
(ii). un radical acétyle, amino, phosphate, nitro, sulfate, carboxy, carboxylique, thiocarboxy, carbamate, thiocarbamate,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique,
(v). un aryle, un naphtyle, un aryl-(alkyle en C1-C30), un(alkyl en C1-C30)-aryle, éventuellement substitués;
   les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
avec les conditions suivantes :
R1, R3 et R5 ne représentant pas simultanément CH₃ dans (IA) et (IB),
R1, R3 et R5 ne représentant pas simultanément CH₂COOH dans (IA), et
R1, R3 et R5 ne représentant pas simultanément CH₂CONHOH dans (IB).

Selon un mode de réalisation avantageux, dans la formule (IA) ou (IB), deux parmi R1, R3 et R5 représentent hydrogène ou méthyle, le troisième étant choisi parmi (vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses.

Selon un autre mode de réalisation avantageux, les para-tertio-butyl calix[6]arènes de l'invention sont des composés de formule (IA) ou (IB) dans laquelle R1, R3 et R5 sont identiques et de préférence ils représentent un hydrogène.

L'invention porte également sur des membranes liquides supportées comprenant un para-tertio-butyl calix[6]arène de formule (IA) ou (IB) 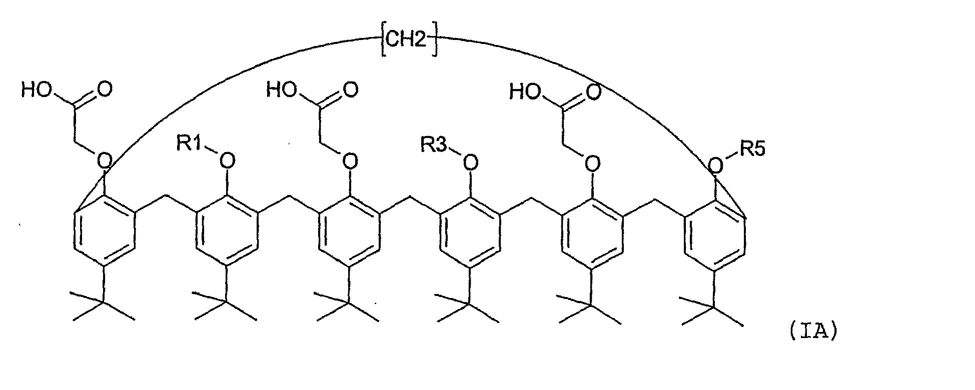 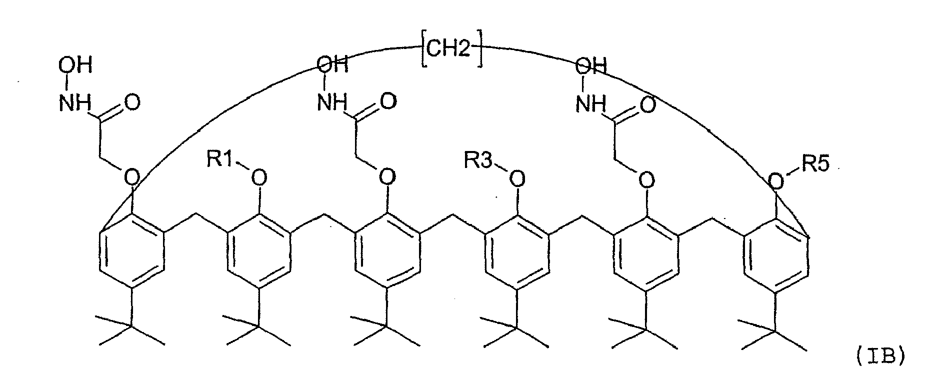 Où R1, R3 et R5, identiques ou différents, représentent, chacun indépendamment :
(i). un atome d'hydrogène, ou d'halogène,
(ii). un radical acétyle, amino, phosphate, nitro, sulfate, carboxy, carboxylique, thiocarboxy, carbamate, thiocarbamate,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique, (v). un aryle, un naphtyle, un aryl-(alkyle en C1-C30), un(alkyl en C1-C30)-aryle, éventuellement substitués;
   les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
ledit produit de formule (IA) ou (IB) étant dissous dans un solvant organique et absorbé sur un support.

Le solvant organique selon l'invention présente un point d'ébullition supérieur à 60 °C, de façon à limiter l'évaporation dudit solvant lors du stockage. Il doit de plus posséder de bonnes propriétés de solubilité vis-à-vis des calixarènes de l'invention. Parmi les solvants les plus intéressants, sans que cette liste soit limitative pour autant, on notera le toluène, le xylène, le chlorobenzène, l'ortho-dichlorobenzène, le nitrobenzène, le 1,4-diisopropyl benzène, l'hexylbenzène, le kérosène, le tétrahydropyrane, le 1,2,3,4-tétrahydronaphtalène, le pentanol et les alcools homologues supérieurs, les glycols et leurs éthers, comme par exemple le diéthylène glycol dibutyl éther, les esters comme le benzoate de méthyle, les éthers comme l'orthonitrophényl pentyléther ou le nitrophényl octyléther.

A titre d'exemple les solubilités en mol/l du composé IA dans différents solvants sont les suivantes :à 25°C 1,2-dichorobenzène : 3.13 10⁻³ M, chlorobutane : 1,6 10⁻³ M, isooctane : 1,25 10⁻³ M, acétate d'isobutyle : 1,75 10⁻³ M, acétate de tertio-butyle : 5,25 10⁻³ M, benzoate d'isoamyle : 2,40 10⁻³ M, acétate de benzyle : 3, 71 10⁻³ M, benzoate de méthyle : 6,41 10⁻³ M, benzonitrile : 1,81 10⁻³ M, 1-hexanol : 17,65 10⁻³ M, 1-heptanol : 14,2810⁻³ M, diéthylène glycol diméthyléther : 23,53 10⁻³ M, diéthylène glycol tertio-butyl éthyléther : 11,34 10⁻³ M, diéthylène glycol dibutyl éther : 19,33 10⁻³ M, dipentyl éther : 2,32 10⁻³ M, isoamyl éther : 2,02 10⁻³ M, isobutyl éther : 1,58 10⁻³ M, 1,1,2-trichloro-trifluoroéthane : 1,57 10⁻³ M, 1,2,3,4-tétrahydronaphtalène : 6,4 10⁻³ M.

Les matériaux supports constituants des membranes liquides supportées de l'invention, sont d'origine minérale choisis dans le groupe comprenant des gels de silice, des oxydes, comme l'alumine, la zircone ou l'oxyde de titane, ou d'origine organique choisis dans le groupe comprenant les polystyrène-divinylbenzènes, les polyéthers, polyacrylamides, polyglycidyl méthacrylates, ou d'origine organo-minérale choisis dans le groupe comprenant les composites silice/dextrane ou hydroxyapatite/agarose, et leurs mélanges.

De préférence le support est sous forme particulaire, la taille de particules variant entre 10 nm et 10 mm, de préférence entre 10 et 50 microns et le diamètre des pores variant entre 10 et 5000 Å, de préférence entre 100 et 500Å.

L'invention porte également sur les membranes liquides contenues dans les membranes liquides supportées, c'est-à-dire sur les para-*tertio*-butyl calix[6]arênes de formules (IA) et (IB), en solution dans un solvant, le solvant étant un solvant organique insoluble dans l'eau tel que décrit ci-dessus.

L'invention concerne également un matériau support qui est un para-*tertio*-butyl calix[6]arène de formule (IIA) ou (IIB) 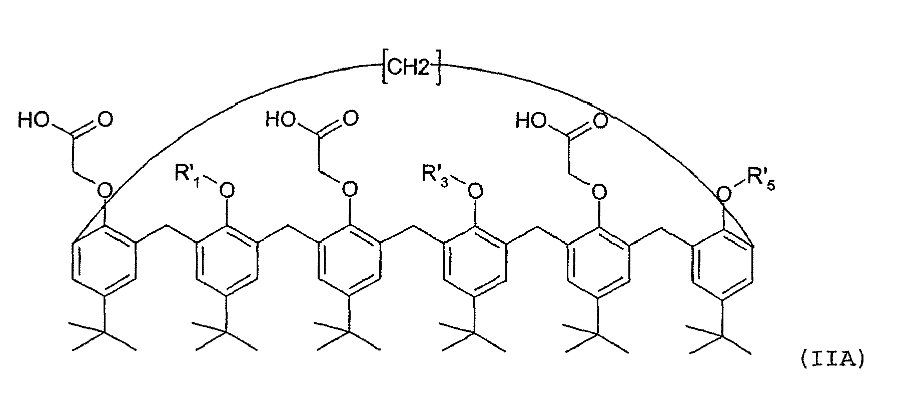 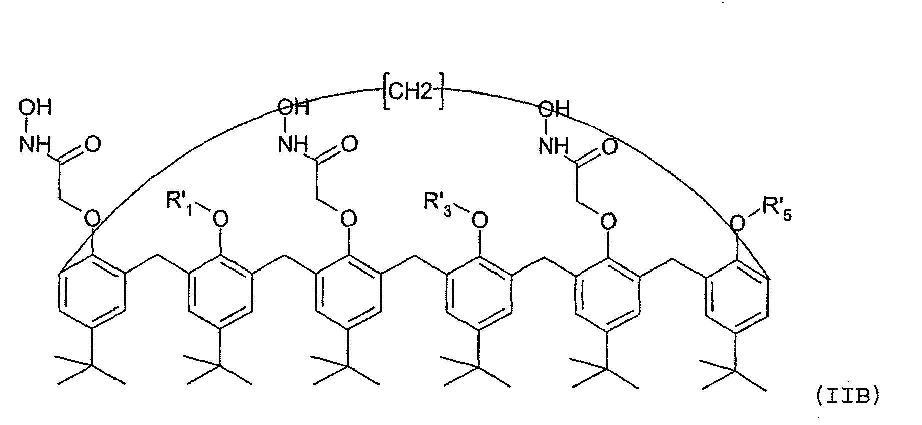 Où R'1, R'3 et R'5, identiques ou différents, représentent, chacun indépendamment :
(i). un atome d'hydrogène, ou d'halogène,
(ii). un radical acétyle, amino, phosphate, nitro, sulfate, carboxy, carboxylique, thiocarboxy, carbamate, thiocarbamate,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique,
(v). un aryle, un naphtyle, un aryl-(alkyle en C1-C30), un(alkyl en C1-C30)-aryle, éventuellement substitués;
   les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
(vii) -ESPACEUR- SUPPORT,
   l'ESPACEUR étant un radical bivalent choisi dans le groupe comprenant les akylènes en C1-C60, de préférence en C1-C30, les (alkyl en C1-C60)-arylènes, les aryl(alkylènes en C1-C60), les aryl(alkyl en C1-C60)-aryles, le radical bivalent pouvant être substitué par des atomes d'halogène, des organométalliques, des fonctions alcools, aminées, acides, esters, carbamates, thiocarbamates, éthers, thiols, époxydes, thioépoxydes, isocyanates, isothiocyanates, ou un carbone de ce radical bivalent pouvant être remplacé par un hétéroatome d'azote, soufre, phosphore, oxygène, bore, arsenic ;
   le SUPPORT étant choisi parmi les supports d'origine minérale ou organique ou organo-minérale, de préférence particulaires, dont la taille de particules varie entre 10 nm et 10 mm, de préférence entre 10 à 50 microns et dont le diamètre des pores varie entre 10 et 5000 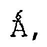 de préférence entre 100 et 500Å;
   à la condition que l'un au moins de R'1, R'3 ou R'5 soit un groupe (vi) ou (vii).

Un matériau support est constitutif de la phase stationnaire d'une colonne chromatographique.

Dans le matériau support de l'invention, le SUPPORT est d'origine minérale choisi dans le groupe comprenant un gel de silice, des oxydes, comme l'alumine, la zircone ou l'oxyde de titane, ou d'origine organique choisi dans le groupe comprenant les polystyrène-divinylbenzène, les polyéthers, polyacrylamides, polyglycidyl méthacrylates ou d'origine organo-minérale choisi dans le groupe comprenant les composites silice/dextrane ou hydroxyapatite/agarose.

Ce SUPPORT est issu d'un support comportant des fonctions chimiques réactives, lesdites fonctions chimiques pouvant être organiques ou minérales, comme par exemple les fonctions chlorure d'acides carboxyliques, aminées, aldéhydes, thiols, sulfochlorure, isocyanate, halogénure métallique, sans que cette liste ne soit limitative pour autant.

A titre d'exemple, des supports particulaires fonctionnalisés sont le copolymère de [5-(4-chlorométhyl)phényl]pentyl-styrène et de divinylbenzène ainsi que le copolymère de [5-(4-bromométhyl)phényl]pentyl-styrène et de divinylbenzène, et le copolymère de 4-chlorométhyl-styrène et de divinylbenzène.

Ils peuvent se présenter sous forme de particules solides sphériques ou irrégulières dont la granulométrie est variable ainsi que la porosité. Différentes qualités commerciales sont disponibles comme les résines Stratospheres^{™} vendues par la société Aldrich.

Pour synthétiser les nouveaux composés de formule générale IA et IB, on procède de la façon suivante :
on synthétise un composé 1,3,5-triméthoxy-*para*-*tertio-*butylcalix[6]arène de formule (a) 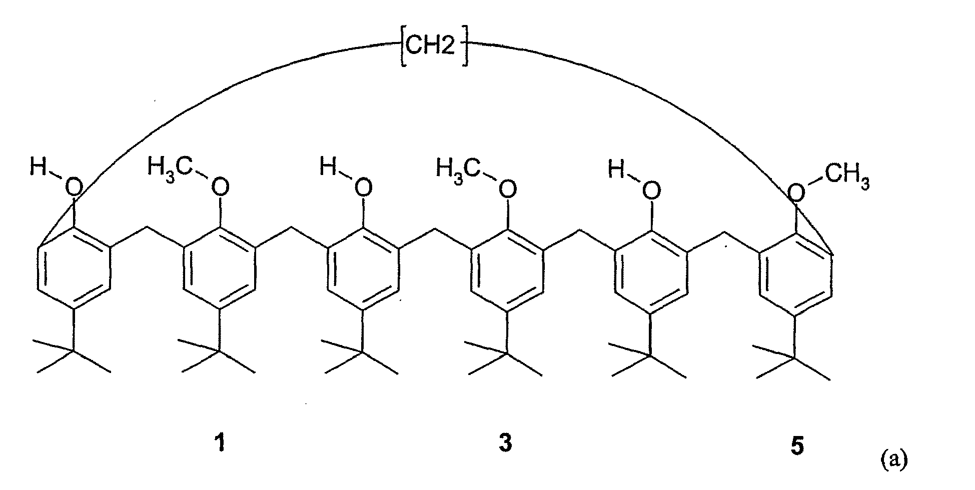
puis un composé précurseur 2,4,6-triéthylester-1,3,5-triméthoxy -*para*-*tertio*-butylcalix[6]arène de formule (b)

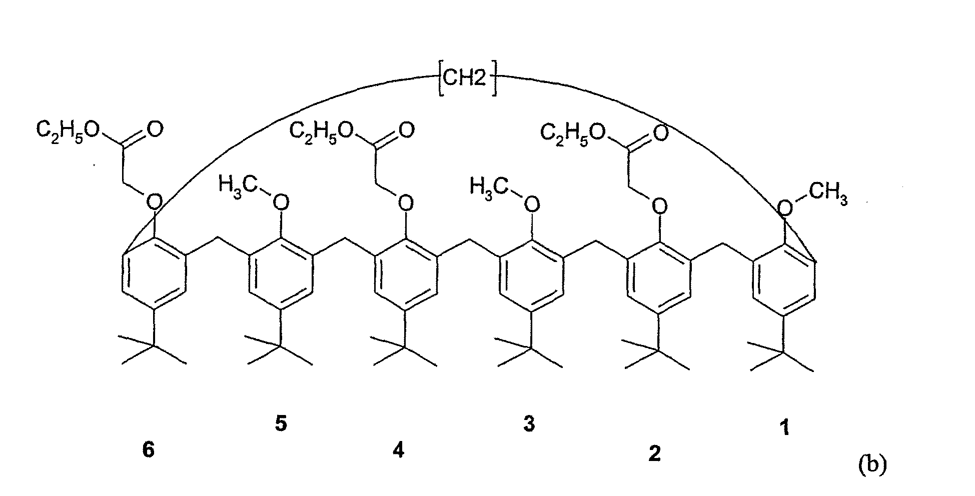

Le composé de formule (b) est ensuite éventuellement subséquemment modifié, par saponification ou par substitution des fonctions ester d'éthyle d'acide carboxylique, pour conduire aux composés de formules A ou B.

Le composé de formule (b) peut également être partiellement ou totalement déméthylé, pour conduire aux composés de formules générales (c1), (c2) ou (c3).

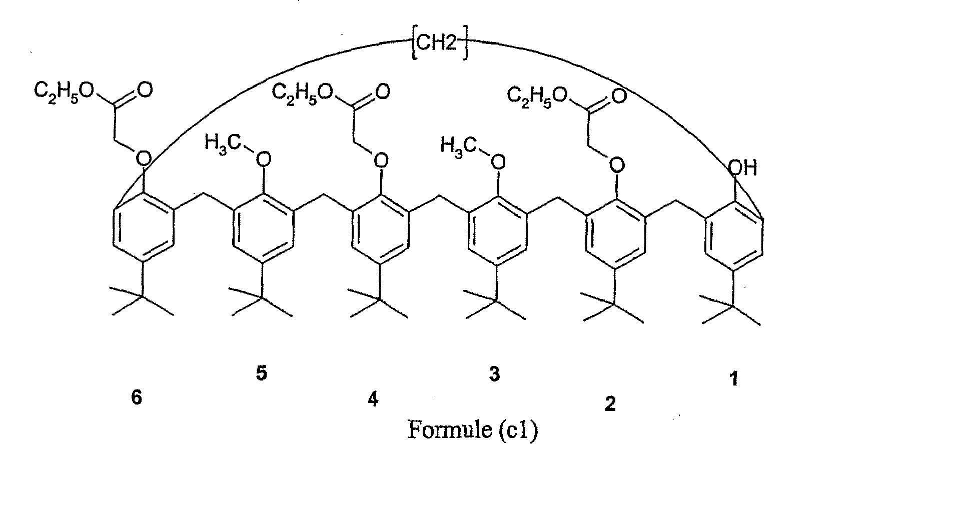

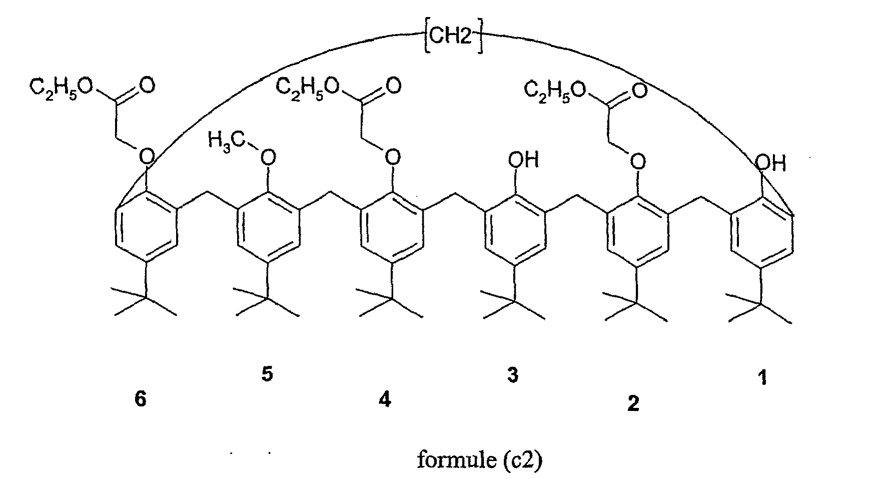

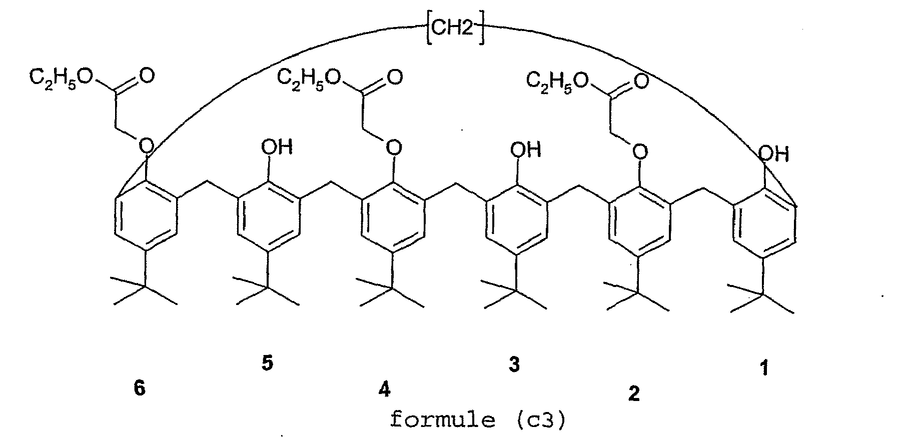

Les composés de formules (c1) ou (c2) ou (c3) sont ensuite modifiés par modification chimique des hydroxyles pour conduire d'une part à des composés de formule générale (IA) par saponification des esters d'éthyle ou d'autre part à des composés de formule générale (IB) par obtention de fonctions acides hydroxamiques à partir des esters d'éthyle.

Selon une variante du procédé, les composés de formules (c1) ou (c2) ou (c3) peuvent être ensuite mis en réaction directe avec des supports convenablement fonctionnalisés, pour conduire à des réactions de greffage covalent via l'activation de leurs hydroxyles phénoliques libres, pour conduire d'une part à des matériaux supports de formule générale (IIA) par saponification des esters d'éthyle ou d'autre part à des matériaux supports de formule générale (IIB) par obtention de fonctions acides hydroxamiques à partir des esters d'éthyle.

Ainsi, un matériau support conforme à l'invention peut également être représenté de la façon suivante :

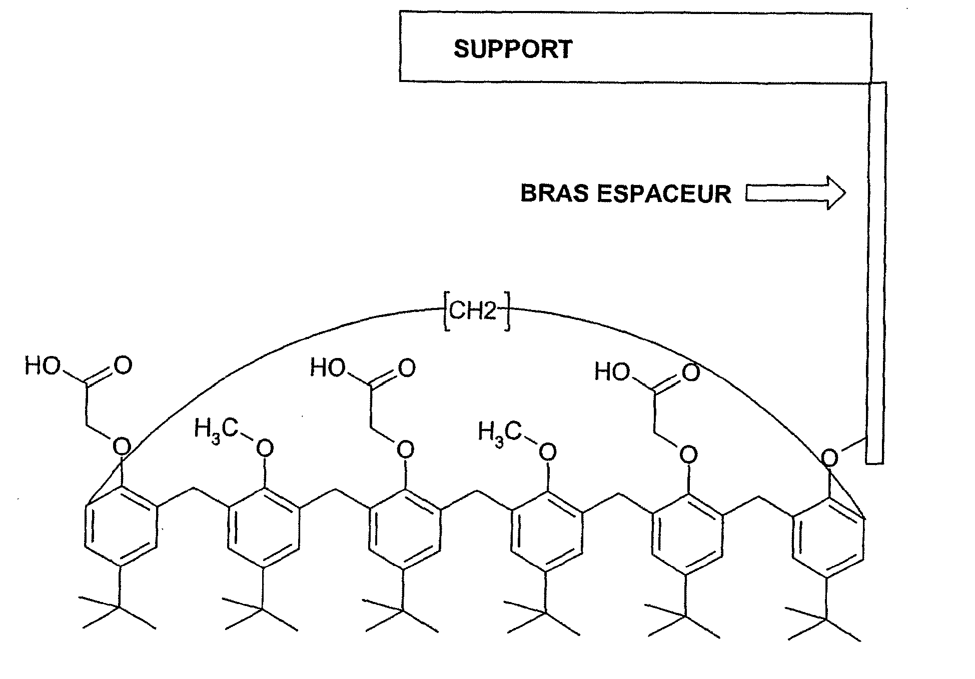

Le détail des étapes de cette synthèse est donnée ci-après :

### A) synthèse de 1,3,5-triméthoxy-para-tertio-butylcalix[6]arène (Mr = 1014)

Le procédé consiste à synthétiser dans un premier temps un précurseur commun comportant des fonctions ester d'acide carboxylique en position 2, 4 et 6 et des fonctions triméthoxy en position 1, 3 et 5.

Dans cet objectif il est d'abord nécessaire de synthétiser un composé symétrique 1,3,5-triméthoxy-*p*-*tert-*butylcalix[6]arène (Mr = 1014) de formule (a) ci-dessus.

Le *para*-*tertio*-butylcalix[6]arène commercial est dissout dans de l'acétone anhydre. Du carbonate de potassium est additionné et la suspension est agitée 3 heures sous azote. De l'iodure de méthyle en excès est alors additionné et la suspension réactionnelle est portée progressivement à reflux pendant 24 heures sous agitation.

L'acétone est ensuite évaporée à sec sous vide d'une pompe laminaire, bain-marie régulé à 60 °C. Le résidu de mise à sec solide obtenu est solubilisé dans du chloroforme. Après dissolution, de l'eau est ajoutée et le milieu biphasique est placé sous vive agitation puis acidifié à l'aide d'acide chlorhydrique concentré 12M. La phase organique inférieure est ensuite récupérée par décantation puis lavée à l'eau jusqu'à neutralité de l'eau de lavage contenue dans la phase supérieure. La phase organique inférieure est ensuite séchée sur sulfate de sodium anhydre, puis filtrée. La phase organique limpide est concentrée à sec, bain-marie à 60 °C sous vide laminaire. Le résidu de mise à sec est ensuite purifié par chromatographie basse pression sur du gel de silice de qualité chromatographique. L'éluant utilisé est le chlorure de méthylène pur de synthèse stabilisé à l'amylène. La pureté des différentes fractions est contrôlée dans chlorure de méthylène/éthanol 95/5 par CCM sur plaques de silice vierge.

Les fractions contenant le composé symétrique 1,3,5-triméthoxy-*p*-*tert*-butylcalix[6] arène mono tache en CCM (révélation par la vapeur d'iode) sont réunies puis concentrées à sec.

Le résidu est utilisé tel quel à l'étape suivante.

### B) synthèse de 2,4,6-triéthylester-1,3,5-triméthoxy,-para-tertio-butylcalix[6]arène (Mr = 1272, 5) (formule (b))

Dans un second temps le procédé consiste à synthétiser le précurseur commun comportant des fonctions ester d'acide carboxylique en positions 2,4 et 6 et des fonctions triméthoxy en positions 1,3 et 5, en modifiant tous les hydroxyles phénoliques du composé obtenu précédemment, et repérés en positions 2,4 et 6, les positions 1,3 et 5 étant protégées par les groupements méthoxy.

Le produit obtenu à l'exemple précédent (formule (a)) est dissous dans le DMF (diméthyl formamide) anhydre (séché sur hydrure de sodium), sous atmosphère d'azote. Un très large excès de carbonate de césium est additionné et la suspension obtenue est agitée 4 heures sous azote. Un très large excès de bromoacétate d'éthyle est ensuite coulé en 5 minutes sur la suspension réactionnelle et le milieu fortement agité est porté progressivement à reflux pendant 24 heures, sous bullage d'azote.

Le DMF est évaporé à sec sous vide d'une pompe laminaire, bain-marié régulé à 80 °C. Le résidu de mise à sec solide obtenu est solubilisé dans du chloroforme. Après dissolution, de l'eau est ajoutée et le milieu biphasique est placé sous vive agitation, puis acidifié avec de l'acide chlorhydrique concentré 12M. La phase organique inférieure est ensuite lavée plusieurs fois à l'eau puis elle est séchée sur sulfate de sodium anhydre. Après filtration, la phase organique est concentrée à sec, bain-marie à 60 °C sous vide laminaire. Le résidu de mise à sec est repris dans de l'éthanol. Une suspension blanche est obtenue. Le solide blanc est récupéré par filtration. Il est lavé plusieurs fois sur le filtre à l'éthanol, puis séché à 40 °C sous vide en étuve.

### C) synthèse de 2,4,6-triéthylester-

1-hydroxy, 3,5-diméthoxy -*para*-*tertio*-butylcalix[6]arène (composé (c1))
1,3-dihydroxy, 5-monométhoxy-*para*-*tertio*-butylcalix[6]arène (composé (c2))
1,3,5-trihydroxy, -*para*-*tertio*-butylcalix[6]arène (composé (c3))

Dans une troisième étape optionnelle le procédé consiste à déméthyler partiellement ou totalement le précurseur commun de formule (b), comportant des fonctions ester d'acide carboxylique en positions 2, 4 et 6 et à la fois des fonctions hydroxyles phénoliques et méthoxy en positions 1, 3 et 5 (composés (c1) ou (c2)), ou uniquement des groupements hydroxyles phénoliques en position 1, 3 et 5 (composé (c3)).

Pour ce faire, le composé de formule (b), obtenu précédemment est dissous dans du chloroforme anhydre préalablement séché sur hydrure de sodium. Le milieu est agité sous atmosphère d'azote puis du triméthylsilyl iodure (agent de déméthoxylation des calixarènes) est additionné, en stoechiométrie ou en défaut selon que l'on veuille privilégier une mono, di ou tri déméthylation, et le milieu réactionnel est porté 2 heures à reflux sous bullage d'azote. La cinétique de la réaction est effectuée par contrôle CCM dans toluène/acétate d'éthyle 90/10 sur plaque silice/polyester. Du triméthylsilyl iodure est éventuellement à nouveau additionné en une seule fois après refroidissement du milieu réactionnel, selon la cinétique de formation des espèces désirées (molécules de formule c1 ou (c2) ou (c3). Celui-ci est à nouveau porté progressivement à reflux pendant 2 heures ou plus.

La réaction est stoppée par addition d'eau. Le milieu réactionnel est acidifié avec HCl 1M et la phase organique inférieure, rouge brique, est récupérée par décantation. Elle est lavée à l'eau jusqu'à pH neutre de l'eau de lavage (phase supérieure).

La phase chloroformique inférieure est évaporée à sec sous vide d'une pompe laminaire, bain-marie régulé à 60 °C après séchage sur du sulfate de sodium anhydre. Le résidu de mise à sec obtenu est ensuite purifié par chromatographie basse pression sur du gel de silice de qualité chromatographique. L'éluant utilisé est le mélange toluène/acétate d'éthyle 90/10. La pureté des différentes fractions est contrôlée dans le mélange toluène/acétate d'éthyle 90/10 par CCM sur plaques de silice vierge déposée sur support de polyester. Les fractions contenant les composés (c1) ou (c2) ou (c3) sont réunies et mises à sec séparément, dans les conditions d'évaporation décrites précédemment.

Chaque résidu de mise à sec est utilisé tel quel pour d'éventuelles modifications chimiques ultérieures.

### D) synthèse de 2,4,6-triéthylester-

1-R1, 3,5-diméthoxy -*para*-*tertio*-butylcalix[6]arène (composé (d1))
1,3-R1-R3, 5-monométhoxy-*para*-*tertio*-butylcalix[6]arène (composé (d2))
1,3,5-R1-R3-R5, -*para*-*tertio*-butylcalix[6]arène (composé (d3))

Dans une autre étape optionnelle le procédé consiste à synthétiser un composé de formule générale (d1) ou (d2) ou (d3) à partir respectivement des composés (c1) ou (c2) ou (c3) obtenus précédemment. Les fonctions hydroxyles phénoliques en positions 1, et/ou 3 et/ou 5 sont modifiées et des groupements R1 et/ou R3 et/ou R5 sont introduits, sachant qu'ils ne peuvent représenter des hydroxyles ou des méthyles.

Pour ce faire, un composé de formule (c1) ou (c2) ou (c3), obtenu précédemment est dissous dans le DMF (diméthyl formamide) anhydre (séché sur hydrure de sodium), sous atmosphère d'azote. Un très large excès de carbonate de césium est additionné et la suspension obtenue est agitée 4 heures sous azote. Un très large excès d'halogénure dont le reste organique représente le groupement R1 ou R3 ou R5, est ensuite coulé en 5 minutes sur la suspension réactionnelle et le milieu fortement agité est porté progressivement à reflux pendant 24 heures, sous bullage d'azote.

Le DMF est évaporé à sec sous vide d'une pompe laminaire, bain-marie régulé à 80 °C. Le résidu de mise à sec solide obtenu est solubilisé dans du chloroforme. Après dissolution, de l'eau est ajoutée et le milieu biphasique est placé sous vive agitation, puis acidifié avec de l'acide chlorhydrique concentré 12M. La phase organique inférieure est ensuite lavée plusieurs fois à l'eau puis elle est séchée sur sulfate de sodium anhydre. Après filtration, la phase organique est concentrée à sec, bain-marie à 60 °C sous vide laminaire. Le résidu de mise à sec est repris dans le chloroforme.

La phase chloroformique inférieure est évaporée à sec sous vide d'une pompe laminaire, bain-marie régulé à 60 °C après séchage sur du sulfate de sodium anhydre. Le résidu de mise à sec obtenu est ensuite purifié par chromatographie basse pression sur du gel de silice de qualité chromatographique. L'éluant utilisé est le mélange toluène/acétate d'éthyle 90/10. La pureté des différentes fractions est contrôlée dans le mélange toluène/acétate d'éthyle 90/10 par CCM sur plaques de silice vierge déposée sur support de polyester. Les fractions contenant le composé (d1) ou (d2) ou (d3) sont réunies et mises à sec séparément, dans les conditions d'évaporation décrites précédemment.

Le résidu de mise à sec est utilisé tel quel pour d'éventuelles modifications chimiques ultérieures.

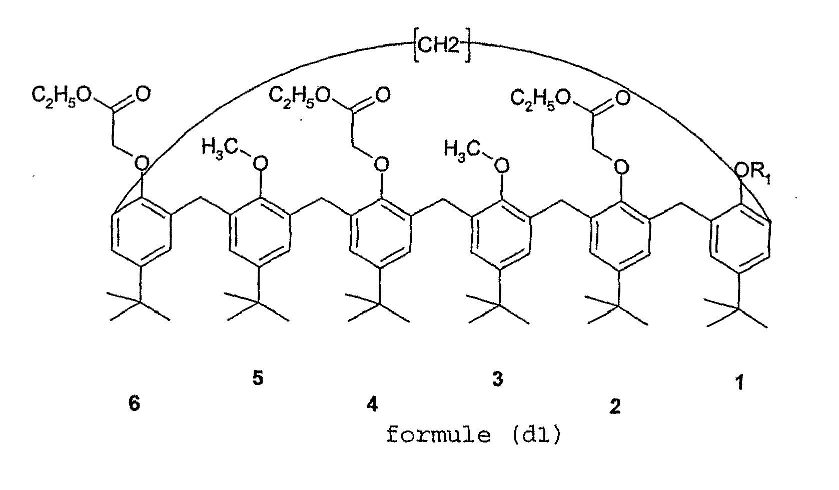

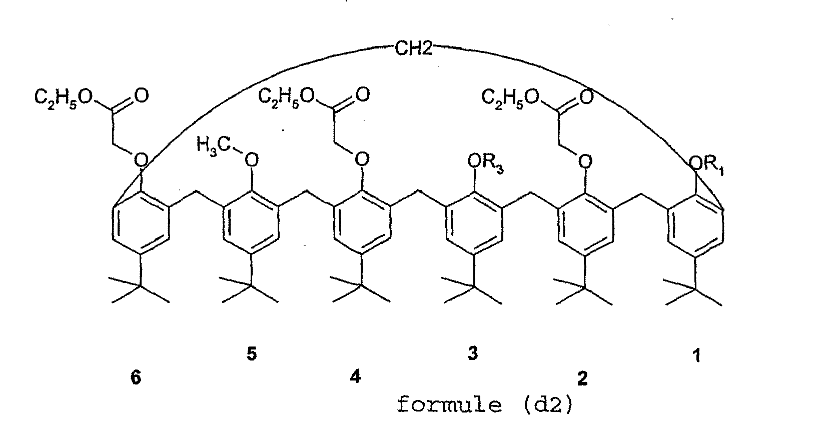

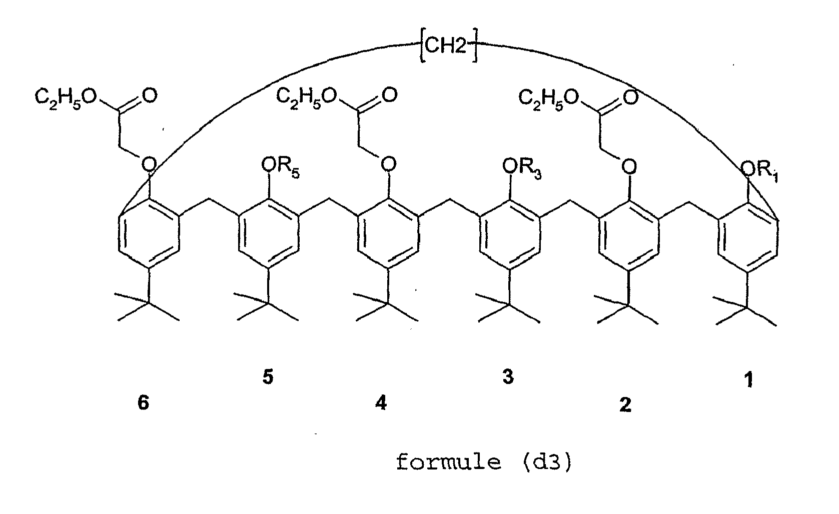

### E) greffage sur support de l'un des composés suivants : (c1), (c2), (c3), (d1), (d2), (d3)

Dans une autre étape optionnelle le procédé consiste à greffer de manière covalente, sur un support, un composé de formule (c1) ou (c2) ou (c3) ou (d1) ou (d2) ou (d3) comportant des fonctions ester d'acide carboxylique en position 2, 4 et 6 et à la fois des fonctions hydroxyles phénoliques et méthoxy en positions 1 et/ou 3 (composés (c1) ou (c3)), ou uniquement des groupements hydroxyles phénoliques en positions 1, 3 et 5 (composé (c3)), ou des fonctions R1 et/ou R3 et/ou R5 (ou R1, R3, R5 sont différents de l'hydrogène et du méthyle), en positions 1, 3 ou 5 (composés (d1), (d2), (d3)).

Un composé de formule (c1) ou (c2) ou (c3) ou (d1) ou (d2) ou (d3) est dissous dans du diméthylformamide anhydre préalablement séché sur hydrure de sodium. Le milieu est agité sous azote jusqu'à dissolution totale. Du carbonate de césium en très large excès est additionné puis une résine commerciale de fonctionnalité connue et quantifiée, ladite fonctionnalité étant choisie pour pouvoir réagir sur un hydroxyle phénolique ou sur un groupement R1 et/ou R3 et/ou R5, et le milieu réactionnel est porté 72 heures à 60°C sous bullage d'azote.

La suspension réactionnelle est filtrée et le solide est lavé au DMF puis à l'acétone puis avec HCl 1M jusqu'à pH acide de l'eau de lavage. La résine est ensuite lavée à l'eau jusqu'à neutralité puis à l'éthanol.

### F) Synthèse d'un composé de formule générale IIA

Dans une option du procédé, on utilise un composé de formule générale (a) ou ((c1)) ou (c2) ou (c3) ou (d1) ou (d2) ou (d3) ou un composé obtenu en utilisant le procédé décrit dans la partie E.

Ledit composé est dissous ou mis en suspension dans l'éthanol. Une solution aqueuse de potasse, en très large excès par rapport à la stoechiométrie comptée sur le nombre de groupements ester d'éthyle à saponifier, est additionnée et le milieu (solution ou suspension) est porté 4 heures à reflux.

La masse réactionnelle est refroidie et le milieu est acidifié à l'HCl 12M.

La suspension est filtrée et le précipité (ou le support) est lavé à l'eau jusqu'à pH neutre des eaux de filtration, puis à l'éthanol.

Le solide est séché à 40°C sous vide jusqu'à poids constant.

### G) Synthèse d'un composé de formule générale IIB

Dans une option du procédé, on utilise un composé de formule générale (a) ou (c1) ou (c2) ou (c3) ou (d1) ou (d2) ou (d3) ou un composé obtenu en utilisant le procédé décrit dans la partie E.

Le dit composé est dissous ou mis en suspension dans du THF. Une solution méthanolique de chlorhydrate d'hydroxylamine, en excès par rapport à la stoechiométrie comptée sur le nombre de groupements ester d'éthyle, est additionnée. Une seconde solution est alors additionnée, comprenant de la potasse écailles préalablement dissoute dans un mélange méthanol/THF et maintenue à +5°C. Le milieu (solution ou suspension) est agité 7 jours à température ambiante, sous atmosphère d'azote.

La masse réactionnelle est évaporée à sec, bain-marie à 60°C sous vide.

Le résidu est repris dans du chlorure de méthylène puis de l'acide acétique est additionné. Le milieu est aguté 4 heures à 20-25°C puis il est mis à nouveau à sec.

La masse réactionnelle est évaporée à sec, bain-marie à 60°C sous vide.

L'invention porte également sur l'utilisation d'une membrane liquide supportée telle que décrite ci-dessus et/ou d'un matériau support selon l'invention pour la complexation sélective et l'analyse des éléments uranium, américium et plutonium ou d'autres radioéléments sous leur forme cationique.

L'invention porte également sur l'utilisation d'une membrane liquide supportée et/ou d'un matériau support selon l'invention pour retirer d'un mélange d'au moins deux constituants, choisis dans le groupe comprenant les molécules organiques, minérales ou organo-minérales, au moins une partie de l'un de ces constituants, ou pour séparer lesdits constituants par une méthode chromatographique.

Les membranes liquides supportées et les matériaux supports de l'invention sont particulièrement adaptés pour la chromatographie d'exclusion. Ils permettent la détection et la séparation de quantités infimes de produit, notamment la séparation de l'uranium et/ou du plutonium et/ou de l'américium à des teneurs de l'ordre de 1mBq/l.

L'invention va être décrite plus en détails ci-après à l'aide des exemples suivants qui sont donnés à titre d'illustration et ne sont pas limitatifs.

### EXEMPLES :

### EXEMPLE 1 :

### Matériau support contenant le-3,5-diméthoxy-2,4,6-triacide carboxylique-p-tert-butylcalix[6]arène

### 1-1 : synthèse de 1,3,5-triméthoxy-p-tert-butylcalix [6] arène (Mr = 1014)

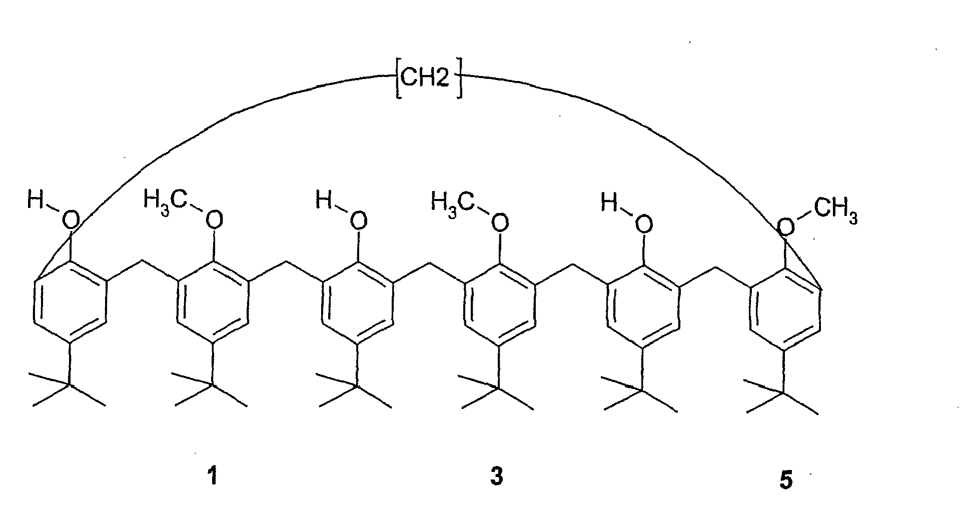

Dans un réacteur en verre de 20 litres, muni d'un condenseur, sont introduits 194,7 g de *p*-*tert*-butylcalix[6]arène (0,2 mol - Mr = 972) et 15 d'acétone anhydre. Le milieu est agité sous azote jusqu'à dissolution totale. 82,9 g de carbonate de potassium (0,6 mol) sont additionnés et la suspension est agitée 3 heures sous azote. 113,6 g d'iodure de méthyle (0,8 mol) sont coulés en 5 minutes et le milieu réactionnel agité est porté progressivement à reflux pendant 24 heures. L'acétone est évaporée à sec sous vide d'une pompe laminaire, bain-marie régulé à 60 °C. Le résidu de mise à sec solide obtenu est solubilisé dans 5,0 1 de chloroforme. Après dissolution, 1,0 1 d'eau sont ajoutés et le milieu biphasique est placé sous vive agitation. 0,1 1 d'acide chlorhydrique concentré 12M sont additionnés doucement en fonction du dégagement de gaz carbonique. La phase organique est ensuite lavée 5 fois par 1,0 1 d'eau puis séchée sur 200 g de sulfate de sodium anhydre. Après filtration, la phase organique est concentrée à sec, bain-marie à 60 °C sous vide laminaire. 250 g de résidu de mise à sec sont obtenus qui sont ensuite purifiés par chromatographie basse pression sur 15 kg de gel de silice 40-200 µm (pore de 60 Å). L'éluant utilisé est le chlorure de méthylène (40 1).

La pureté des différentes fractions est contrôlée dans chlorure de méthylène/éthanol 95/5 par CCM sur plaques de silice vierge.

40 g de résidu, issu des fractions contenant le 1,3,5-triméthoxy calixarène pur, sont obtenus (39,45 mmol : rendement = 19,7 %).

Le spectre RMN du proton dans CDC13 donne les déplacements chimiques suivants :
7,00 ppm (s, 6H, ArH méta OH) - 6,90 ppm (s, 6H, ArH méta OCH₃) - 6,75 ppm (s, 3H, OH) - 3,89 ppm (s, 9H, OCH₃) - 3,47 ppm (s, 12H, ArCH₂Ar), 1,20 ppm (s, 27H, tert-butyl para OH) - 1,00 ppm (s, 27H, tert-butyl para OCH₃).

### 1-2 : synthèse de 2,4,6-triéthylester-1,3,5-triméthoxy-p-tert-butylcalix[6]arène (Mr = 1272,5)

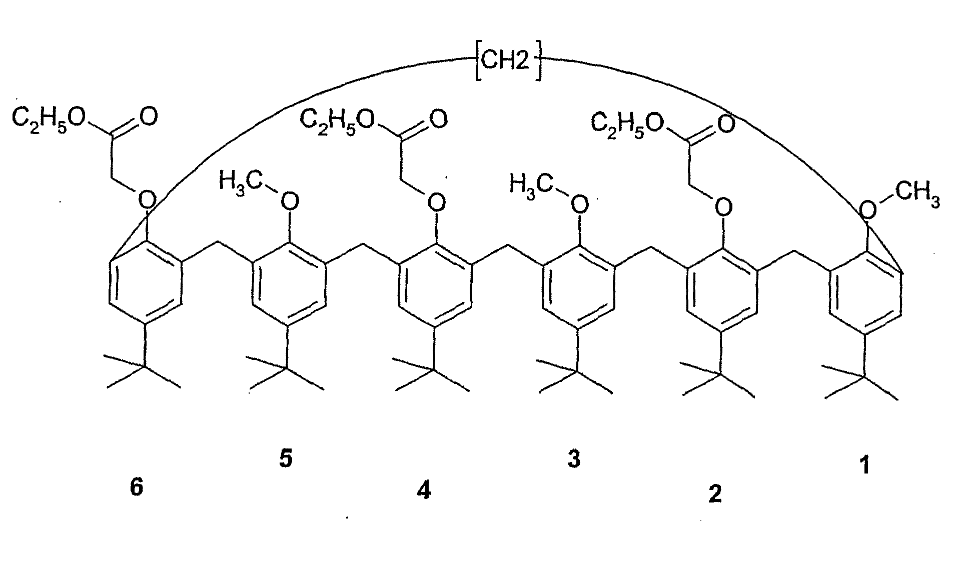

Dans un réacteur en verre de 10 litres, muni d'un condenseur, sont introduits 40,0 g de produit obtenu en 1-1 (39,45 mmol) et 4 1 de diméthyl formamide (DMF) anhydre (séché sur hydrure de sodium). Le milieu est agité sous azote jusqu'à dissolution totale. 81,6 g de carbonate de césium (0,25 mol) sont additionnés et la suspension est agitée 4 heures sous azote. 52,7 g de bromoacétate d'éthyle (0,31 mol) sont coulés en 5 minutes et le milieu réactionnel agité est porté progressivement à reflux pendant 24 heures, sous bullage d'azote.

Le DMF est évaporé à sec sous vide d'une pompe laminaire, bain-marie régulé à 80 °C. Le résidu de mise à sec solide obtenu est solubilisé dans 2,0 1 de chloroforme. Après dissolution, 0,5 1 d'eau sont ajoutés et le milieu biphasique est placé sous vive agitation. 20 ml d'acide chlorhydrique concentré 12M sont additionnés doucement en fonction du dégagement de gaz carbonique. La phase organique est ensuite lavée 5 fois par 0,5 1 d'eau puis séchée sur 20 g de sulfate de sodium anhydre. Après filtration, la phase organique est concentrée à sec, bain-marie à 60 °C sous vide laminaire. Le résidu est repris par 300 ml d'éthanol. Une suspension blanche est obtenue. Le solide est récupéré par filtration et lavages par 3 fois 40 ml d'éthanol, puis séché à 40°C sous vide en étuve.
43,2 g de poudre blanche sont obtenus après séchage à poids constant (33,95 mmol : rendement = 86 %).

Le spectre RMN du proton dans CDCl₃ donne les déplacements chimiques suivants :
6,71 ppm (s large, 12H, ArH méta) - 4,55 ppm (s, 6H, ArCH₂CO₂R) - 4,29 ppm (qt, 6H, O-CH₂-méthyl J=7Hz) - 3,47 ppm (s, 21H, ArCH₂Ar + méthyl OCH₃), 1,38 ppm (s, 54H, tert-butyl) - 1,33 ppm (t, 9H, méthyl OCH₂CH₃ J=7Hz).

### 1-3 : synthèse de 1-hydroxy-3,5-diméthoxy-2,4,6-triéthylester -p-tert-butylcalix [6] arène (Mr = 1258, 5)

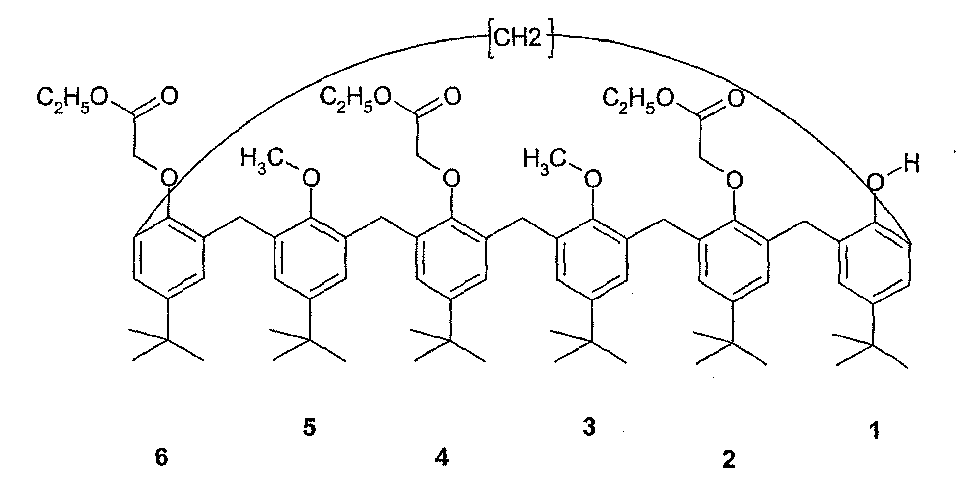

Dans un réacteur verre de 5 litres, muni d'un condenseur, sont introduits 27,8 g de produit (poudre blanche) obtenu en 1-2 ci-dessus (21,85 mmol - Mr = 1272,5) et 1,5 1 de chloroforme anhydre préalablement séché sur hydrure de sodium. Le milieu est agité sous azote jusqu'à dissolution totale. 3,1 ml (4,37 g) de triméthylsilyl iodure (21, 85 mmol - Mr = 200,1) sont additionnés et le milieu réactionnel est porté 2 heures à reflux sous bullage d'azote. La cinétique de la réaction est effectuée par contrôle CCM dans toluène/acétate d'éthyle 90/10 sur plaque silice/polyester. 3,1 ml (4,37 g) de triméthylsilyl iodure sont à nouveau additionnés en une seule fois après refroidissement du milieu réactionnel. Celui-ci est à nouveau porté progressivement à reflux pendant 2 heures.

La réaction est stoppée par addition de 2 1 d'eau. 100 ml d'HCl 1M sont additionnés et la phase organique rouge brique est récupérée. Elle est lavée par 2 fois 1 1 d'eau. La phase chloroformique est évaporée à sec sous vide d'une pompe laminaire, bain-marie régulé à 60°C après séchage par 200 g de sulfate de sodium anhydre. 27,2 g de résidu de mise à sec sont obtenus qui sont ensuite purifiés par chromatographie basse pression sur 3 kg de gel de silice 40-200 µm (pore de 60 Å). L'éluant utilisé est un mélange toluène/acétate d'éthyle 90/10 (20 1). La pureté des différentes fractions est contrôlée dans le mélange toluène/acétate d'éthyle 90/10 par CCM sur plaques de silice vierge déposée sur support de polyester.

8,5 g de résidu sont obtenus (6,75 mmol : rendement=30,7%), après mise à sec bain-marie à 60 °C sous vide des fractions contenant le 1-hydroxy-3,5-diméthoxy-2,4,6-triéthylester - *p*-tert-butylcalix[6]arène pur.

Le spectre de masse réalisé en FAB par ionisation chimique positive confirme la présence du produit attendu (MH+ à 1259 daltons).

Le spectre RMN du proton dans CDCl₃ donne les déplacements chimiques suivants :
6,75 ppm (s, 13H, ArH méta + OH phénol) - 4,55 ppm (s, 6H, ArCH₂CO₂R) - 4,29 ppm (qt, 6H, O-CH₂-méthyl J=7Hz) - 3,47 ppm (s, 20H, ArCH₂Ar + méthyl OCH₃), 1,38 ppm (s, 54H, tert-butyl) - 1,33 ppm (t, 9H, méthyl OCH₂CH₃).

### 1-4 : Matériau support contenant le-3,5-diméthoxy-2,4,6-triéthylester -p-tert-butylcalix [6] arène

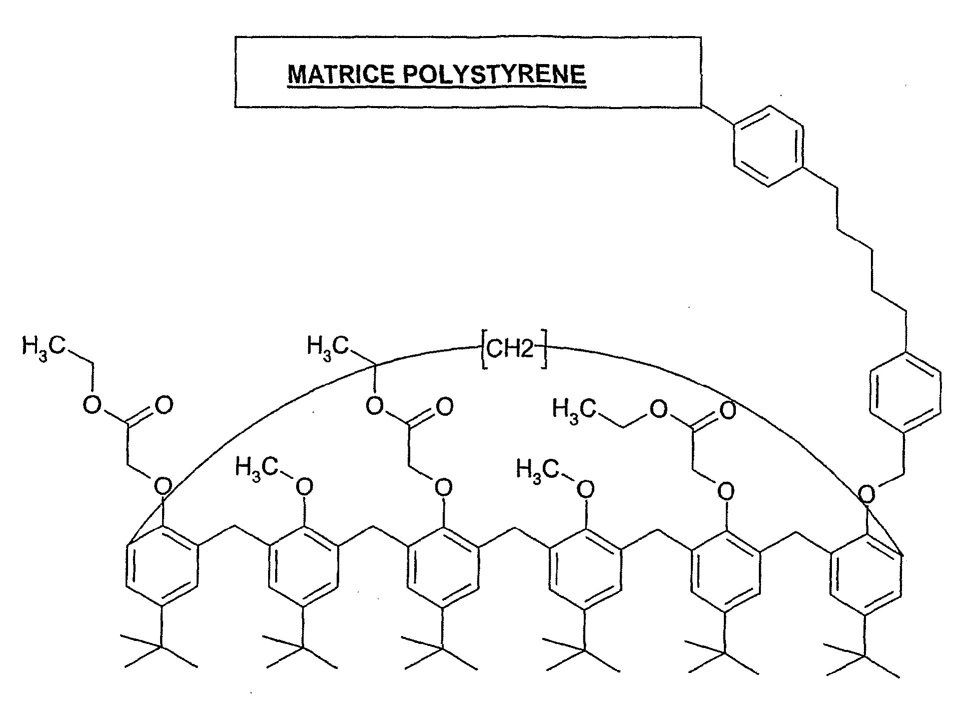

Dans un réacteur en verre de 250 ml, muni d'un condenseur, sont introduits 8,5 g de produit obtenu en 1-3 ci-dessus (6,75 mmol - Mr = 1258,5) et 150 ml de DMF anhydre préalablement séché sur hydrure de sodium. Le milieu est agité sous azote jusqu'à dissolution totale. 20 g de carbonate de césium (61 mmol) sont additionnés puis 10 g de résine commerciale polystyrène modifiée chlorométhylphényl-pentyl (acheté chez Aldrich CMPP rein : [5-[4-(chloromethyl)phenyl]pentyl]styrene, polymer bound référence 513776) et le milieu réactionnel est porté 72 heures à 60°C sous bullage d'azote.

La suspension réactionnelle est filtrée et le solide est lavé au DMF (2 fois 50 ml) puis à l'acétone (3 fois 50 ml) puis avec HCl 1M (4 fois 100 ml) puis à l'eau (5 fois 100 ml) puis à l'éthanol (3 fois 50 ml).

16,15 g de résine sèche sont obtenus, après séchage à 60 °C sous vide jusqu'à poids constant.

Son taux de greffage calculé d'après la microanalyse est de 0,2 mmol de calix/g de résine.

La microanalyse est la suivante :
C % : 80,80 H % : 7,29 Cl % : 0, 79

La microanalyse de la résine chlorométhylphényl-pentyl de départ était la suivante :
C % : 86,52 H % : 7,87 Cl % : 3,91

### 1-5 : Matériau support contenant le-3,5-diméthoxy-2,4,6-triacide carboxylique -p-tert-butylcalix[6]arène

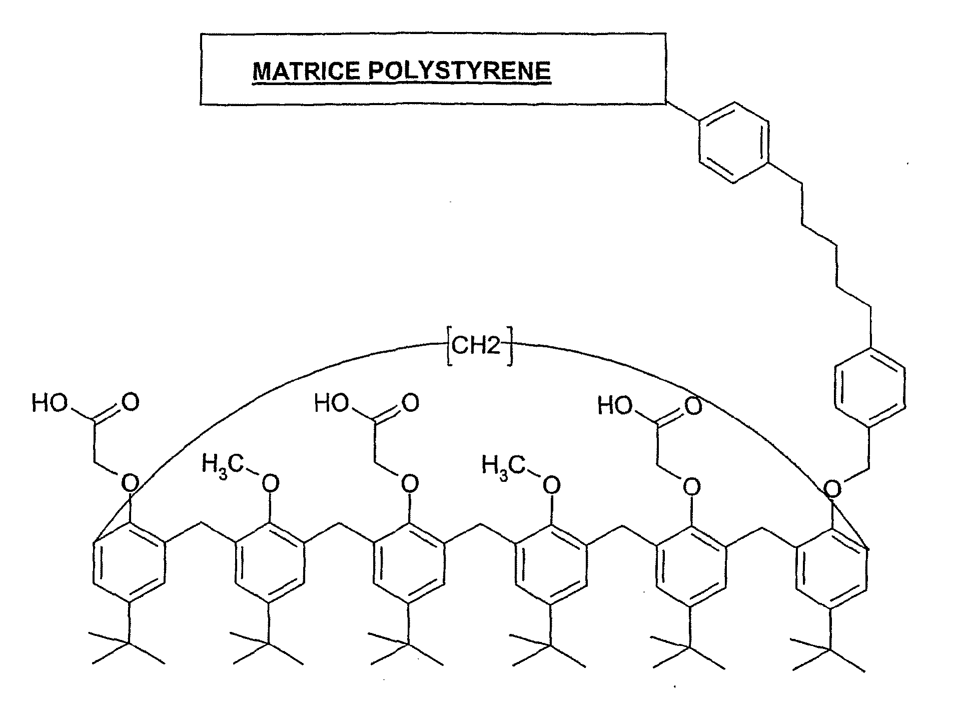

Dans un réacteur en verre de 250 ml, muni d'un condenseur, sont introduits 10 g de résine sèche obtenue en 1-4 ci-dessus et 100 ml d'éthanol. 6,1 g de potasse pastilles à 85 % sont dissous dans 100 ml d'eau et la solution obtenue est additionnée en une seule fois dans le réacteur. Le milieu réactionnel est porté 4 heures à reflux sous bullage d'azote.

La suspension réactionnelle est refroidie puis 12 ml de HCl 12M sont additionnés. Le pH de la suspension est de 1. Après 1 heure d'agitation, la suspension est filtrée puis lavée par 8 fois 100 ml d'eau puis à l'éthanol (3 fois 50 ml).

9,7 g de résine sont obtenus après séchage à 60 °C sous vide jusqu'à poids constant.

La résine est utilisée telle quelle sans caractérisation analytique supplémentaire.

### EXEMPLE 2 :

### Membrane liquide supportée contenant le 1-3,5-triméthoxy-2,4,6-triacide hydroxamique-p-tert-butylcalix[6]arène (Mr = 1234,6)

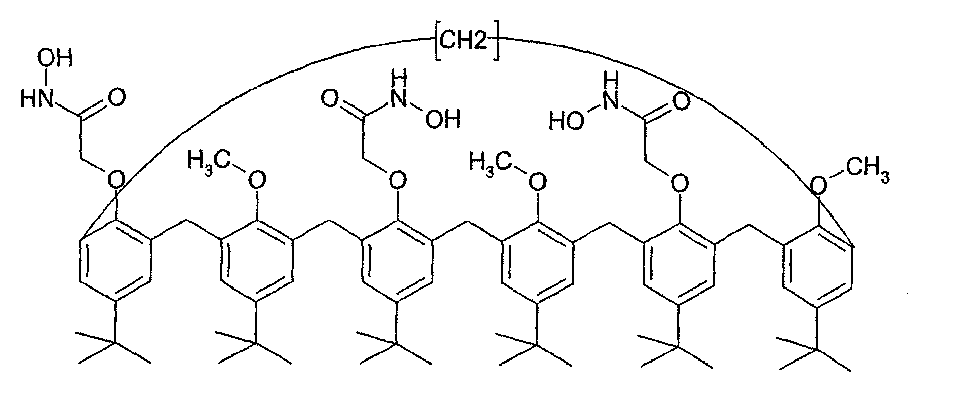

Dans un réacteur verre de 250 ml, muni d'un condenseur, sont introduits 1,8 g de produit obtenu à l'exemple 1-2 (1,4 mmol) et 50 ml de tétrahydrofurane (THF). 2,02 g de chlorhydrate d'hydroxylamine (29 mmol) sont dissous dans 80 ml de méthanol et 40 ml de THF, puis la solution obtenue est additionnée sur celle contenue dans le réacteur. Ensuite une autre solution préalablement préparée (et maintenue à + 5 °C) de potasse écailles (2,04 g à 100 %, soit 36 mmol) dans 24 ml de méthanol et 12 ml de THF, est additionnée en une seule fois dans le réacteur. Le milieu réactionnel est agité 7 jours à température ambiante sous bullage d'azote.

La suspension réactionnelle est évaporée à sec, bain-marie à 60 °C, sous vide. Le résidu est repris dans le mélange chlorure de méthylène/acide acétique 50 ml/10 ml et agité pendant 4 heures. La masse réactionnelle est mise à sec, bain-marie à 60 °C, sous vide.

Le résidu de mise à sec est repris dans 20 ml de dichlorométhane. Le produit attendu précipite sous forme d'un solide blanc. 1,5 g de solide blanc sont obtenus après séchage à 60 °C sous vide jusqu'à poids constant (rendement = 85,9 %).

Le spectre de masse réalisé en ESI électrospray confirme la présence du produit attendu (M/z à 1234 daltons).

Le spectre RMN du proton à 300 MHz dans le DMSO donne les déplacements chimiques suivants :
10,8 ppm (s, 3H, -NH) - 9,08 ppm (s, 3H, -OH d'hydroxamique) - 7,23 ppm (s, 6H, ArH méta OCH₂COOEt) - 6,57 ppm (s, 6H, ArH méta OCH₃) - 4,44 à 4,33 ppm (q, 18H, ArCH₂CO₂R + ArCH₂Ar ) - 2,50 ppm (s large, 9H, méthoxy) - 1,36 ppm (s, 27H + tert-butyl para OCH₂CONHOH), 0,73 ppm (s, 27H, tert-butyl para OCH₃).
2g de résine Macroprep époxy de chez BIORAD, granulométrie de 70 à 100 µm, lot 11/99, sont additionnés à la solution préalablement préparée de 15,6 mg de solide blanc obtenu précédemment prélevés sur les 1,5 g de solide blanc obtenu précédemment dissous dans 20 ml de dichlorométhane et 1,12 ml de 1,2,3,4-tétrahydronaphtalène. La suspension est évaporée doucement à température ambiante jusqu'à poids constant : obtention d'un solide blanc.
Poids obtenu : 2,2 g

### EXEMPLE 3 :

### Membrane liquide supportée contenant le 1-3,5-triméthoxy-2,4,6-triacide carboxylique -p-tert-butylcalix[6]arène (Mr = 1174,4)

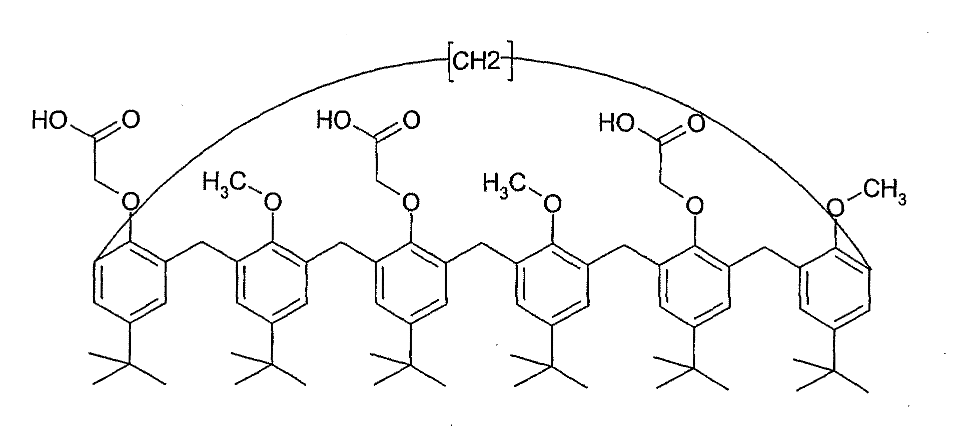

Dans un réacteur en verre de 250 ml, muni d'un condenseur, sont introduits 5,9 g de produit (poudre blanche) obtenu à l'exemple 1-2 (4,00 mmol) et 150 ml d'éthanol. Une solution préalablement préparée (et maintenue à + 5 °C) de potasse écailles (12 g à 100 %, soit 214 mmol) dans 150 ml d'eau, est additionnée en une seule fois dans le réacteur. Le milieu réactionnel est porté 4 heures à reflux sous bullage d'azote. 25 ml d'HCl 12M sont additionnés lentement après refroidissement de la masse réactionnelle à 20 °C. Le produit attendu précipite sous forme d'un solide blanc. La suspension est ensuite filtrée et le solide est lavé par 8 fois 50 ml d'eau puis 2 fois 50 ml d'éthanol. Le solide est ensuite séché à 40 °C sous vide jusqu'à poids constant.
4,2 g de solide blanc sont obtenus après séchage à 40 °C sous vide jusqu'à poids constant (rendement = 99 %).

Le spectre RMN du proton à 300 MHz dans le CDCl₃ donne les déplacements chimiques suivants :
6,97 ppm (s, 6H, ArH méta OCH₂COOH) - 6,94 ppm (s, 6H, ArH méta OCH₃) - 3,84 ppm (s, 6H, ArCH₂COOH) - 3,73 ppm (s large, 9H, méthoxy) - 1,12 ppm (s, 27H + tert-butyl para OCH₂COOH), 1,09 ppm (s, 27H, tert-butyl para OCH₃).
2 g de résine Macroprep époxy de chez BIORAD, granulométrie de 70 à 100 µm, lot 11/99, sont additionnés à la solution préalablement préparée de 12,5 mg prélevés sur les 4,2 g de solide blanc obtenu précédemment dissous dans 20 ml de dichlorométhane et 1,12 ml de 1,2,3,4-tétrahydronaphtalène. La suspension est évaporée doucement à température ambiante jusqu'à poids constant. Obtention d'un solide blanc.
Poids obtenu : 2,2 g

### EXEMPLE 4 :

### Complexation et extraction sélective de l'américium

Dans cet exemple, on fixe, au moyen des calixarènes de l'invention, l'américium présent à une concentration de 10⁻¹¹ mol.l⁻¹ dans une solution aqueuse de NaNO₃ 0,04 mol.l⁻¹ simulant le milieu urinaire, et ajustée à pH = 4.

Pour cette expérience, on utilise 100 mg de matériau support de l'exemple 1-5 conditionné en colonne.

Une solution de NaNO₃ 0,04 mol.l⁻¹ à pH = 4 est passée sur la colonne pour se trouver dans les conditions optimales d'extraction (étape de conditionnement). La solution aqueuse contenant l'américium est ensuite passée sur la colonne (étape de fixation). Une solution de NaNO₃ 0,04 mol.l⁻¹, à pH = 4 est de nouveau passée afin d'éliminer l'américium non extrait par le calixarène (étape de rinçage). L'américium fixé est enfin élué par une solution HNO₃ 2M (étape d'élution). Les solutions s'écoulent par gravitation naturelle. L'américium est mesuré par spectrométrie alpha dans chaque solution en pied de colonne. Les résultats des mesures permettent de calculer le rendement de fixation et le rendement d'élution de l'américium.

Les résultats de cette expérience sont donnés dans le tableau I ci-après.

### EXEMPLE 5 :

### Complexation et extraction sélective de l'uranium

Dans cet exemple, on fixe, au moyen des calixarènes de l'invention, l'uranium présent à une concentration de 10⁻⁸ mol.l⁻¹ dans une solution aqueuse de NaNO₃ 0,04 mol.l⁻¹ simulant le milieu urinaire, et ajustée à pH = 4.

Pour cette expérience, on utilise 1 g de membrane liquide supportée de l'exemple 2 conditionnée en colonne.

Les étapes de conditionnement, fixation et rinçage sont identiques à celles décrites dans l'exemple 4. L'uranium fixé est élué par une solution HNO₃ 1M. L'uranium est mesuré par spectrométrie alpha ou par spectrométrie de masse (ICP-MS) dans chaque solution en pied de colonne. Les résultats des mesures permettent de calculer le rendement de fixation et le rendement d'élution de l'uranium.

Les résultats obtenus sont donnés dans le tableau I ci-après.

### EXEMPLE 6 :

### Complexation et extraction sélective de l'uranium dans l'urine

Dans cet exemple, on fixe, au moyen des calixarènes de l'invention, l'uranium présent à une concentration de 5.10⁻⁶ g/l dans de l'urine.

Pour cette expérience, on utilise 1 g de membrane liquide supportée de l'exemple 3 conditionnée en colonne.

Une étape préalable de minéralisation de l'urine est réalisée par chauffage par micro-ondes. Le résidu de minéralisation est repris par une solution HNO₃ 2M, puis le pH de la solution est ajusté à 4 avant le passage sur colonne.

Les étapes de conditionnement, fixation, rinçage et élution sont identiques à celles décrites dans l'exemple 4. L'uranium est mesuré et les résultats sont exprimés comme précédemment.

Les résultats obtenus sont donnés dans le tableau I ci-après.

### EXEMPLE 7 :

### Complexation et extraction sélective du plutonium

Dans cet exemple, on fixe, au moyen des calixarènes de l'invention, le plutonium présent à une concentration de 10⁻¹⁰ mol.l⁻¹ dans une solution aqueuse de NaNO₃ 0,04 mol.l⁻¹ simulant le milieu urinaire, et ajustée à pH = 4.

Pour cette expérience, on utilise 100 mg de matériau support de l'exemple 1-5 conditionné en colonne.

Les étapes de conditionnement, fixation, rinçage et élution sont identiques à celles décrites dans l'exemple 4. Le plutonium est mesuré par spectrométrie alpha ou par spectrométrie de masse (ICP-MS) dans chaque solution en pied de colonne. Les résultats des mesures permettent de calculer le rendement de fixation et le rendement d'élution du plutonium.

Les résultats obtenus sont donnés dans le tableau I ci-dessous.

**Tableau 1 : Rendements de fixation et d'élution (%)**

| | Rendement de fixation (%) | Rendement d'élution (%) |
|---|---|---|
| Exemple 4 | 97 ± 1 | 94 ± 9 |
| Exemple 5 | 99 ± 1 | 93 ± 1 |
| Exemple 6 | 98 ± 1 | 102 ± 3 |
| Exemple 7 | 83 ± 1 | 87 ± 6 |

### EXEMPLE 8 :

### Membrane liquide supportée contenant le 1-hydroxy-3,5-diméthoxy-2,4,6-triacide carboxylique -p-tert-butylcalix[6]arène (Mr = 1174,4)

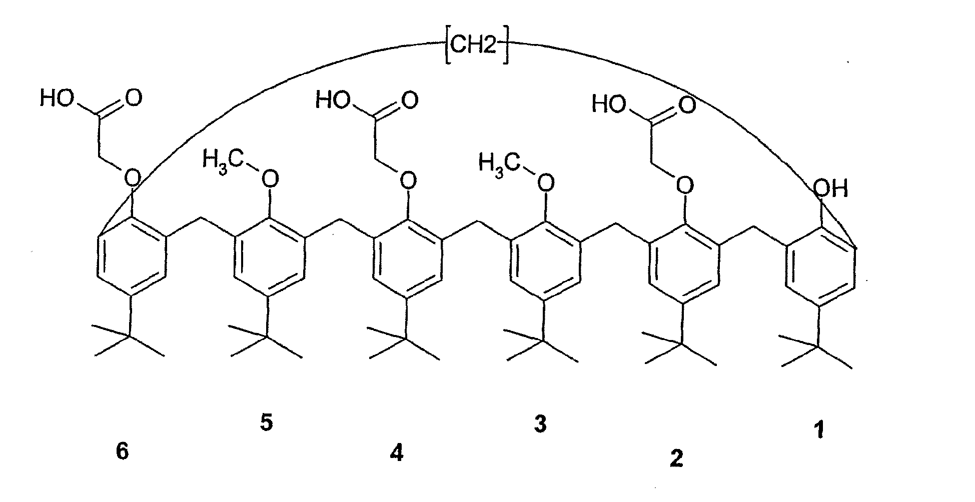

Dans un réacteur en verre de 250 ml, muni d'un condenseur, sont introduits 5,03 g de produit (poudre blanche) obtenu à l'exemple 1-3, lors de la mise en oeuvre d'un second essai identique au précédent, (4,00 mmol) et 150 ml d'éthanol. Une solution préalablement préparée (et maintenue à + 5 °C) de potasse écailles (12 g à 100 %, soit 214 mmol) dans 150 ml d'eau, est additionnée en une seule fois dans le réacteur. Le milieu réactionnel est porté 4 heures à reflux sous bullage d'azote. 25 ml de HCl 12M sont additionnés lentement après refroidissement de la masse réactionnelle à 20 °C. Le produit attendu précipite sous forme d'un solide blanc. La suspension est ensuite filtrée et le solide est lavé par 8 fois 50 ml d'eau puis 2 fois 50 ml d'éthanol. Le solide est ensuite séché à 40 °C sous vide jusqu'à poids constant.
4,65 g de solide blanc sont obtenus après séchage à 40 °C sous vide jusqu'à poids constant (rendement = 99 %).

Le spectre RMN du proton à 300 MHz dans le CDCl₃ donne les déplacements chimiques suivants :
6,86 ppm (s, 13 H, ArH méta + OH phénol) - 3,95 ppm (s, 6H, ArCH₂COOH) - 3,73 ppm (s large, 6H, méthoxy) - 1,12 ppm (s, 27H + tert-butyl para OCH₂COOH), 1,09 ppm (s, 27H, tert-butyl para OCH₃).
2 g de résine Macroprep époxy de chez BIORAD, granulométrie de 70 à 100 µm, lot 11/99, sont additionnés à la solution préalablement préparée de 12,5 mg prélevés sur les 4,65 g de solide blanc obtenu précédemment dissous dans 20 ml de dichlorométhane et 1,12 ml de 1-heptanol. La suspension est évaporée doucement à température ambiante jusqu'à poids constant. Obtention d'un solide blanc.
Poids obtenu : 2,2 g

### Complexation et extraction sélective de l'uranium

Dans cet exemple, on fixe, au moyen des calixarènes de l'invention, l'uranium présent à une concentration de 10⁻⁸ mol.l⁻¹ dans une solution aqueuse de NaNO₃ 0,04 mol.l⁻¹ simulant le milieu urinaire, et ajustée à pH = 4.

Pour cette expérience, on utilise 1 g de membrane liquide supportée obtenue ci-dessus, conditionnée en colonne.

Les étapes de conditionnement, fixation, rinçage et élution sont identiques à celles décrites dans l'exemple 4. L'uranium est mesuré et les résultats sont exprimés comme dans les exemples précédents.

Les résultats obtenus sont donnés dans le tableau II ci-après.

### Complexation et extraction sélective du thorium

Dans cet exemple, on fixe, au moyen des calixarènes de l'invention, le thorium présent à une concentration de 10⁻⁸ mol.l⁻¹ dans une solution aqueuse de NaNO₃ 0,04 mol.l⁻¹ simulant le milieu urinaire, et ajustée à pH = 3.

Pour cette expérience, on utilise 1 g de la membrane liquide supportée obtenue ci-dessus, conditionnée en colonne.

Les étapes de conditionnement, fixation, rinçage et élution sont identiques à celles décrites dans l'exemple 4, les solutions de conditionnement et de rinçage étant ajustées à pH 3. Le thorium est mesuré par spectrométrie alpha ou par spectrométrie de masse (ICP-MS) dans chaque solution en pied de colonne et les résultats sont exprimés comme dans les exemples précédents.

Les résultats obtenus sont donnés dans le tableau II ci-dessous.

**Tableau II : Rendements de fixation et d'élution (%)**

| Exemple 8 | Rendement de fixation (%) | Rendement d'élution (%) |
|---|---|---|
| uranium | 99 ± 1 | 73 ± 8 |
| thorium | 60 ± 1 | 70 ± 1 |

## Revendications

1. Para-*tertio*-butyl calix[6]arène de formule (IA) ou (IB) 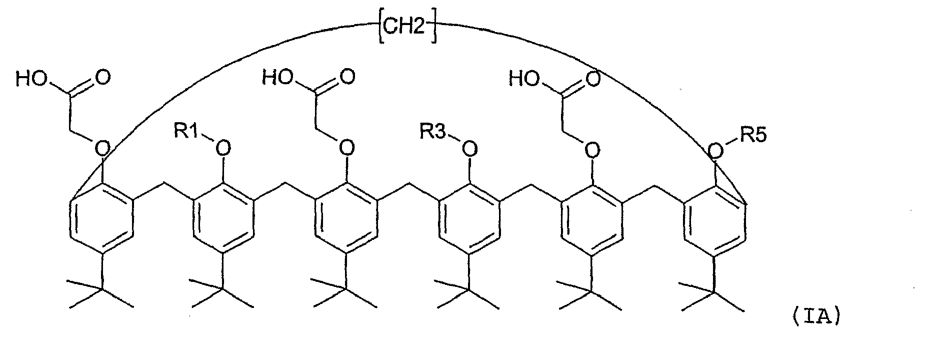 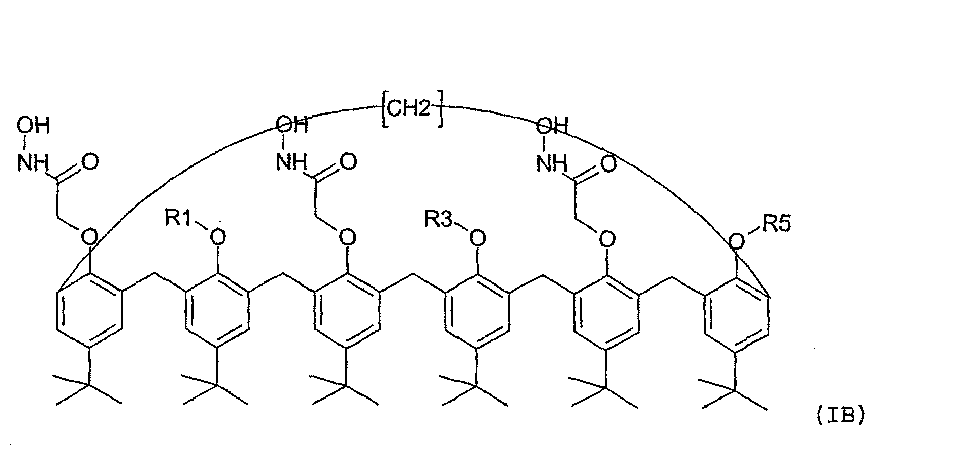 Où R1, R3 et R5, identiques ou différents, représentent, chacun indépendamment :
(i). un atome d'hydrogène, ou d'halogène,
(ii). un radical acétyle, amino, phosphate, nitro, sulfate, carboxy, carboxylique, thiocarboxy, carbamate, thiocarbamate,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique,
(v). un aryle, un naphtyle, un aryl-(alkyle en C1-C30), un(alkyl en C1-C30)-aryle, éventuellement substitués;
les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
avec les conditions suivantes :
R1, R3 et R5 ne représentant pas simultanément CH₃ dans (IA) et (IB),
R1, R3 et R5 ne représentant pas simultanément CH₂COOH dans (IA), et
R1, R3 et R5 ne représentant pas simultanément CH₂CONHOH dans (IB).

2. Para-tertio-butyl calix[6]arène selon la revendication 1, **caractérisé par le fait que** deux parmi R1, R3 et R5 représentent hydrogène ou méthyle, le troisième étant choisi parmi (vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses.

3. Para-*tertio*-butyl calix[6]arène selon la revendication 1, **caractérisé par le fait que** R1, R3 et R5 sont identiques.

4. Para-tertio-butyl calix[6]arène selon la revendication 1, **caractérisé par le fait que** R1, R3 et R5 représentent un hydrogène.

5. Membrane liquide supportée comprenant un para-tertio-butyl calix[6]arène de formule (IA) ou (IB) 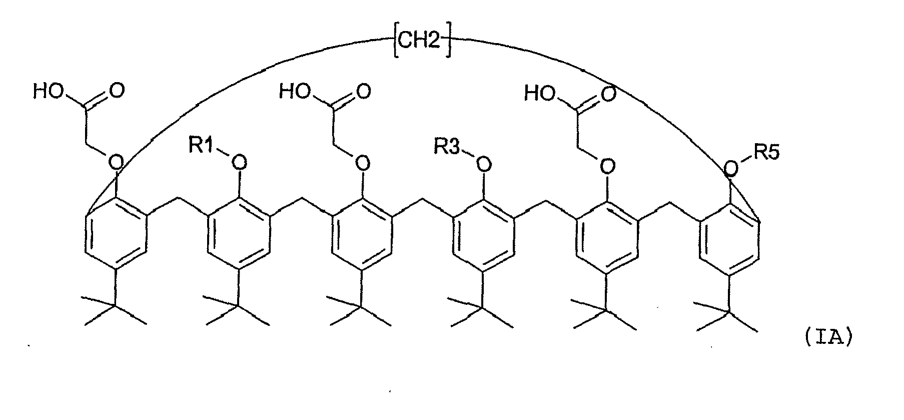 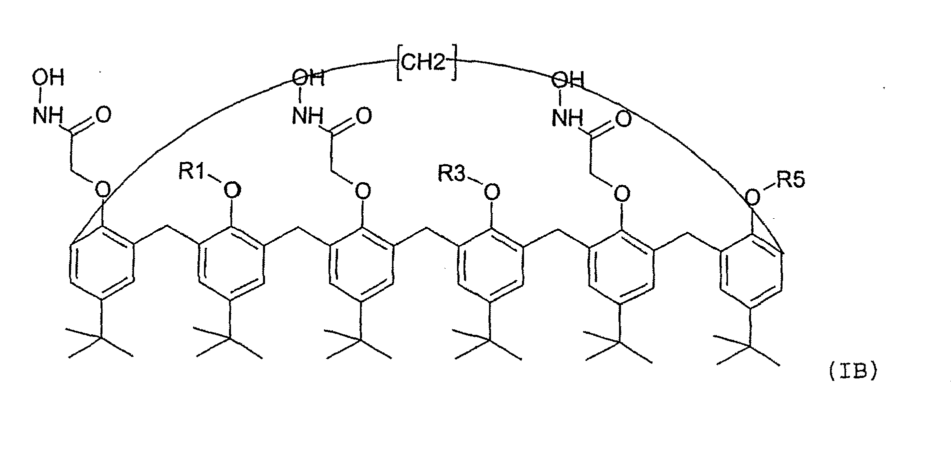 où R1, R3 et R5, identiques ou différents, représentent, chacun indépendamment :
(i). un atome d'hydrogène, ou d_{'}halogène,
(ii). un radical acétyle, amino, phosphate, nitro, sulfate, carboxy, carboxylique, thiocarboxy, carbamate, thiocarbamate, ,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique,
(v). un aryle, un naphtyle, un aryl-(alkyle en C1-C30), un(alkyl en C1-C30)-aryle, éventuellement substitués;
les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi) un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
ledit produit de formule (IA) ou (IB) étant dissous dans un solvant organique et absorbé sur un support.

6. Membrane liquide supportée comportant un para *tertio-*butyl calix[6]arène selon l'une quelconque des revendications 2 à 4.

7. Membrane liquide supportée selon la revendication 5 ou 6, **caractérisée par le fait que** le solvant organique présente un point d'ébullition supérieur à 60 °C et est choisi dans le groupe comprenant notamment le toluène, le xylène, le chlorobenzène, l'ortho-dichlorobenzène, le nitrobenzène, le 1,4-diisopropyl benzène, l'hexylbenzène, le kérosène, le tétrahydropyranne, le 1,2,3,4-tétrahydronaphtalène, le pentanol et les alcools homologues supérieurs, les glycols et leurs éthers, comme par exemple le diéthylène glycol dibutyl éther, les esters comme le benzoate de méthyle, les éthers comme l'orthonitrophényl pentyléther ou le nitrophényl octyléther, et leurs mélanges.

8. Membrane liquide supportée selon l'une quelconque des revendications 5 à 7, **caractérisée par le fait que** le support est un support d'origine minérale choisi dans le groupe comprenant un gel de silice, des oxydes, comme l'alumine, la zircone ou l'oxyde de titane, ou d'origine organique choisi dans le groupe comprenant les polystyrène-divinylbenzène, les polyéthers, polyacrylamides, polyglycidyl méthacrylates, ou d'origine organo-minérale choisi dans le groupe comprenant les composites silice/dextrane ou hydroxyapatite/agarose, et leurs mélanges.

9. Membrane liquide supportée selon l'une quelconque des revendications 5 à 8, **caractérisée par le fait que** le support est un support particulaire dont la taille de particules varie entre 10 nm et 10 mm, de préférence entre 10 et 50 microns et dont le diamètre des pores varie entre 10 et 5000 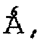 de préférence entre 100 et 500Å.

10. Matériau support qui est un para-*tertio*-butyl calix[6]arène de formule (IIA) ou (IIB) 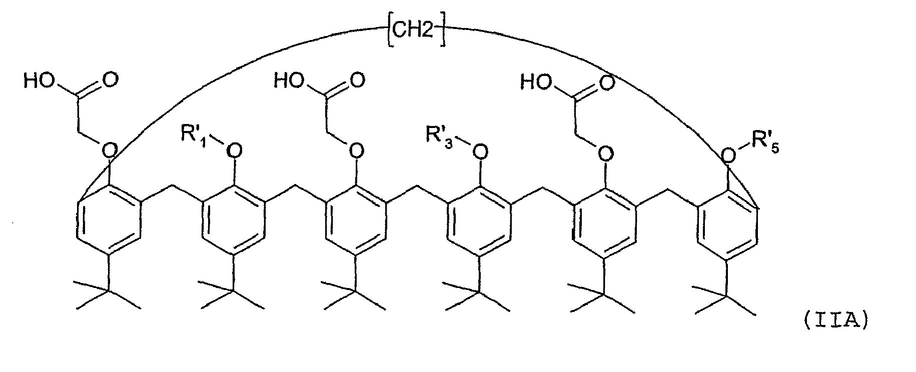 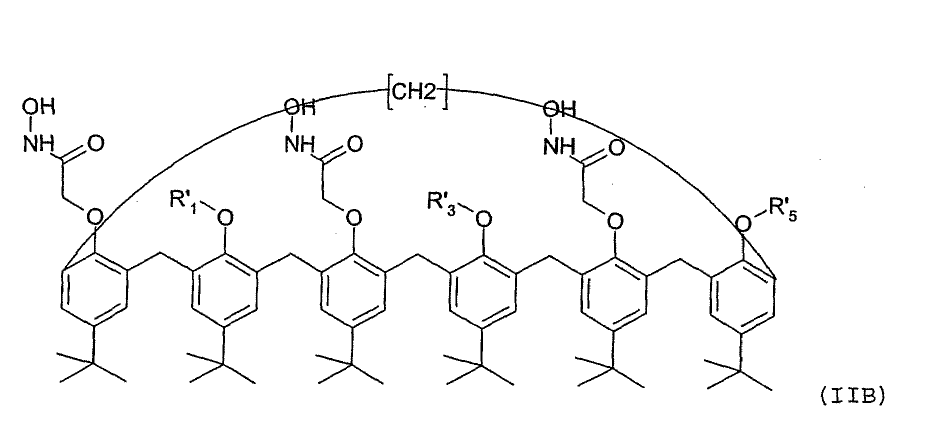 Où R'1, R'3 et R'5, identiques ou différents, représentent, chacun indépendamment :
(i). un atome d'hydrogène, ou d'halogène,
(ii). un radical acétyle, amino, phosphate, nitro, - sulfate, carboxy, carboxylique, thiocarboxy, carbamate, thiocarbamate, ,
(iii). un alkyle linéaire ou ramifié, ayant 1 à 60, de préférence 1 à 30 atomes de carbone, éventuellement substitué, présentant éventuellement au moins une insaturation éthylénique ou acétylénique,
(iv). un cycloalkyle ayant de 3 à 12 atomes de carbone, éventuellement substitué, présentant éventuellement au moins une insaturation éthylénique ou acétylénique,
(v). un aryle, un naphtyle, un aryl-(alkyle en C1-C30), un(alkyl en C1-C30)-aryle , éventuellement substitués;
les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;
(vi). un polymère choisi dans le groupe comprenant les polystyrènes, les copolymères de chloro- et/ou bromométhyl-styrène et de divinylbenzène, les polyéthers, les polyacrylamides, les polyglycidyl méthacrylates, les dextranes, et les agaroses ;
(vii) -ESPACEUR- SUPPORT,
l'ESPACEUR étant un radical bivalent choisi dans le groupe comprenant les akylènes en C1-C60, de préférence en C1-C30, les (alkyl en C1-C60)-arylènes, les aryl(alkylènes en C1-C60), les aryl(alkyl en C1-C60)-aryles, le radical bivalent pouvant être substitué par des atomes d'halogène, des organométalliques, des fonctions alcools, aminées, acides, esters, carbamates, thiocarbamates, éthers, thiols, époxydes, thioépoxydes, isocyanates, isothiocyanates, ou un
carbone de ce radical bivalent pouvant être remplacé par un hétéroatome d'azote, soufre, phosphore, oxygène, bore, arsenic ;
le SUPPORT étant choisi parmi les supports d'origine minérale ou organique ou organo-minérale, de préférence particulaires, dont la taille de particules varie entre 10 nm et 10 mm, de préférence entre 10 à 50 microns et dont le diamètre des pores varie entre 10 et 5000 Ǻ, de préférence entre 100 et 500Å;
à la condition que l'un au moins de R'1, R'3 ou R'5 soit un groupe (vi) ou (vii).

11. Matériau support selon la revendication 10, **caractérisé par le fait que** le SUPPORT est un support d'origine minérale choisi dans le groupe comprenant des gels de silice, des oxydes, comme l'alumine, la zircone ou l'oxyde de titane, ou d'origine organique choisi dans le groupe comprenant les polystyrène-divinylbenzènes, les polyéthers, polyacrylamides, polyglycidyl méthacrylates ou d'origine organo-minérale choisi dans le groupe comprenant les composites silice/dextrane ou hydroxyapatite/agarose.

12. Utilisation d'une membrane liquide supportée selon l'une quelconque des revendications 5 à 9 et/ou d'un matériau support selon la revendication 10 ou 11, pour la complexation sélective et l'analyse des éléments uranium, américium et plutonium ou d'autres radionéléments sous leur forme cationique.

13. Utilisation d'une membrane liquide supportée selon l'une quelconque des revendications 5 à 9, et/ou d'un matériau support selon la revendication 10 ou 11, pour retirer d'un mélange d'au moins deux constituants, choisis dans le groupe comprenant les molécules organiques, minérales ou organo-minérales, au moins une partie de l'un de ces constituants, ou pour séparer lesdits constituants par une méthode chromatographique.
